# EUROPEAN PATENT APPLICATION

(11) **EP 4 321 145 A1**
(43) Date of publication of application: **14.02.2024**
(21) Application number: 23186010.7
(22) Date of filing: 18.07.2023
(51) Int. Cl.: A61K 6/40, A61K 6/77, A61K 6/889

(54) **DENTAL RESIN-REINFORCED GLASS IONOMER CEMENT KIT**

(30) Priority: 19.07.2022 JP 2022115161
(71) Applicant: Shofu Inc., Kyoto-shi, Kyoto 605-0983 (JP)
(72) Inventor: UCHIDA, Jun, Kyoto, 605-0983 (JP); SAKAMOTO, Shuji, Kyoto, 605-0983 (JP); TSUKAMOTO, Masahiro, Kyoto, 605-0983 (JP)
(74) Representative: Eisenführ Speiser

(57) **Abstract**

[Problem]

To provide a dental resin-reinforced glass ionomer cement kit which can exhibit high adhesive property to tooth substance by a simple procedure without complicated operations and contains a dental tooth surface treatment material composition and a dental resin-reinforced glass ionomer cement composition.

[Solution]

To provide a dental resin-reinforced glass ionomer cement kit comprising a dental tooth surface treatment material composition (i) and a dental resin-reinforced glass ionomer cement composition (ii), wherein
the dental tooth surface treatment material composition (i) contains
(A) water-soluble organic solvent: 20 % by mass or more and 79 % by mass or less,
(B) water: 20 % by mass or more and 79 % by mass or less, and
(C) polymerizable monomer having acid anhydride structure: 1 % by mass or more and 30 % by mass or less, and

the dental resin-reinforced glass ionomer cement composition (ii) contains
(D) acid-reactive glass powder,
(B) water,
(E) polymer of acidic group-containing polymerizable monomer,
(F) tri or more functional (meth)acrylamide-based polymerizable monomer,
(G) hydroxyl group-containing (meth)acrylate-based polymerizable monomer, and
(H) polymerization initiator.

## Description

### BACKGROUND OF THE INVENTION

### Field of the Invention

The present invention relates to a dental resin-reinforced glass ionomer cement kit for repairing a tooth in which a form was partially lost by mainly caries, breakages and the like or for adhering or luting a dental prosthesis device to a tooth in which a form was partially lost.

### Description of the Related Art

In a dental practice, in order to restore aesthetically and functionally the tooth in which a form was partially lost by caries, breakages and the like, a direct restoration in which a dental composite resin for filling or a dental glass ionomer cement filling is filled into the tooth and an indirect restoration in which a dental prosthesis device is adhered or luted to a tooth by using a dental adhesive resin cement or a dental glass ionomer cement for luting has been performed.

In general, a dental resin-based material, represented by a dental filling composite resin and a dental adhesive resin cement, has been widely used in recent years because it has high mechanical property and excellent aesthetic property due to high transparency, and has an advantage in operability. However, a lot of dental resin-based materials have no self-adhesive property to tooth substances, and when these materials are used, it is necessary to use a dental primer and/or a dental bonding material simultaneously.

In contrast, the dental glass ionomer cement (for filling or luting) exhibits self-adhesive property to the tooth substance by action of the polycarboxylic acid in the components. Therefore, one of advantage is that it is not necessary to use a dental bonding material or a dental primer simultaneously. In addition, because a fluoride ion is sustained release from the hardened body, the protective effect of the second caries can be expected. On the other hand, the dental glass ionomer cement has lower mechanical properties as compared with the dental resin-based material, and therefore it is often used to repair areas that are difficult to apply strong stress. In addition, a dental glass ionomer cement is opaque and includes a problem in aesthetic property.

On the other hand, in order to compensate for the respective disadvantages of the dental resin-based material and the dental glass ionomer cement, a dental resin-reinforced glass ionomer cement in which the component composition of the dental resin-based material and the component composition of the dental glass ionomer cement are combined has been proposed.

The dental resin-reinforced glass ionomer cement has the advantages of both the dental glass ionomer cement and the dental resin-based material, and has a long-term fluoride ion sustained release ability, and transparency and mechanical property exceeding conventional dental glass ionomer cements. Further, when the photocurability is given, because the composition can be cured at the timing intended by the operator by irradiation with light, unlike the conventional dental glass ionomer cement, there is an advantage that there is no need to wait for hardening and the like.

Further, examples of an additional feature of the dental resin-reinforced glass ionomer cement include self-adhesive property to tooth substance. However, there is a case where it is desirable to reliably improve the adhesive property to tooth substance, such as restoration of the occlusal surface of the posterior tooth on which occlusal force is applied. In such case, tooth surface treatment may be performed prior to applying the dental resin-reinforced glass ionomer cement to the restoration site.

Japanese Unexamined Patent Application Publication No. JPH 8-217612 A discloses a tooth surface treatment material that dissolves and removes a smear layer (tooth chippings) serving as an adhesion inhibitor by remaining on the tooth surface after forming a cavity and.

Japanese Unexamined Patent Application Publication No. JPH 9-249514 A discloses a tooth surface treatment material which modifies a hydrophilic tooth surface including a smear layer to be somewhat hydrophobic to improve affinity between the tooth substance and the dental resin-reinforced glass ionomer cement having intermediate properties between hydrophobicity and hydrophilicity.

### SUMMARY OF THE INVENTION

### Technical Problem

The tooth surface treatment material of Japanese Unexamined Patent Application Publication No. JPH 8-217612 A contains an aqueous solution of polycarboxylic acid as a main component, and is used by applying to the tooth surface after cavity formation, leaving for a certain period of time, washing with water, and drying. By applying this tooth surface treatment material, the smear layer dissolves in the tooth surface treatment material and is removed when washed with water, so that the adhesive property of the dental resin-reinforced glass ionomer cement to the tooth substance is improved. However, in this type of tooth surface treatment material, it is necessary to sufficiently wash it away with water after applying to the tooth surface, and therefore the operation is complicated.

The tooth surface treatment material of Japanese Unexamined Patent Application Publication No. JPH 9-249514 A contains an organic compound having a phosphoric acid group or the like, (meth)acrylate-based polymerizable monomer, and water as a main component, and is used by applying to the tooth surface after cavity formation, leaving for a certain period of time, and drying without washing with water. By applying this tooth surface treatment material, the smear layer dissolves and the (meth)acrylate polymerizable monomer penetrates the tooth surface while incorporating the smear layer, so that the affinity between the polymerizable monomer contained in the dental resin-reinforced glass ionomer cement and the tooth surface is improved to improve the adhesive property.

However, in order to further improve the adhesive property of this type of tooth surface treatment material to tooth structure, it has been suggested that it is necessary to impart polymerization curability to the tooth surface treatment material by adding a chemical polymerization initiator in the case of two-component type, or adding a photopolymerization initiator in the case of one-component type. However, the operation is complicated because it is required in the former case to mix the two liquids and to wait time until hardening, and it is required in the latter case to irradiate light. Furthermore, since this type of tooth surface treatment material contains a (meth)acrylate-based polymerizable monomer in an acidic aqueous solution, there is a problem that the viscosity increases over time by gradually progressing polymerization of acrylic acid or methacrylic acid having high polymerization activity which is generated by hydrolyzing of the ester bond of the (meth)acryloyloxy group.

Therefore, an object of the present invention is to provide a dental resin-reinforced glass ionomer cement kit which can exhibit high adhesive property to tooth substance by a simple procedure without complicated operations and contains a dental tooth surface treatment material composition and a dental resin-reinforced glass ionomer cement composition. Another object of the present invention is to provide a dental tooth surface treatment material composition which is excellent in storage stability and can be used with the same operational feeling for a long period of time.

### Solution to Problem

As a result of intensive studies by the present inventor to solve the above problems, it has been found that, a dental tooth surface treatment material composition containing a polymerizable monomer having an acid anhydride structure and water as main components has excellent storage stability, and a combination of the dental tooth surface treatment material composition with a dental resin-reinforced glass ionomer cement composition containing a tri or more functional (meth)acrylamide-based polymerizable monomer can exhibit a specific high adhesive property to the tooth substance even if the tooth surface treatment procedure is a very simple, and the present invention has been completed.

That is, the present invention provides a dental resin-reinforced glass ionomer cement kit comprising a dental tooth surface treatment material composition (i) and a dental resin-reinforced glass ionomer cement composition (ii), wherein
the dental tooth surface treatment material composition (i) contains
   (A) water-soluble organic solvent: 20 % by mass or more and 79 % by mass or less,
   (B) water: 20 % by mass or more and 79 % by mass or less, and
   (C) polymerizable monomer having acid anhydride structure: 1 % by mass or more and 30 % by mass or less, and
the dental resin-reinforced glass ionomer cement composition (ii) contains
   (D) acid-reactive glass powder,
   (B) water,
   (E) polymer of acidic group-containing polymerizable monomer,
   (F) tri or more functional (meth)acrylamide-based polymerizable monomer,
   (G) hydroxyl group-containing (meth)acrylate-based polymerizable monomer, and
   (H) polymerization initiator.

Further, the present invention also provides a method for applying the dental resin-reinforced glass ionomer cement kit to tooth substance, comprising
a first step in which the dental tooth surface treatment material composition (i) is applied to the tooth surface,
a second step in which the applied surface is dried immediately without washing with water,
a third step in which the dried surface is applied with the dental resin-reinforced glass ionomer cement composition (ii) without irradiating light, and
a fourth step in which the applied dental resin-reinforced glass ionomer cement composition (ii) is hardened by irradiating light.

### Advantageous Effects of Invention

The dental resin-reinforced glass ionomer cement kit of the present invention can improve the adhesive property of the dental resin-reinforced glass ionomer cement composition to the tooth substance by only simple operation without complicated operations such as a standing step, a water washing step and a polymerization step after application of the dental tooth surface treatment material composition to the tooth surface. In addition, the dental tooth surface treatment material composition constituting the dental resin-reinforced glass ionomer cement kit of the present invention undergoes little change in properties over time, and can be used for a long period of time with the same feeling of operation.

### DETAILED DESCRIPTION OF THE PREFERRED EMBODIMENTS

The present invention will be described in detail below. In the present specification, "water-soluble" means that the solubility in water at 25 °C is 3% by mass or more.

In the present specification, the term "(meth)acrylate" inclusively refers to both acrylate and methacrylate, the term "(meth)acryloyl" inclusively refers to both acryloyl and methacryloyl, the term "(meth)acrylic acid" inclusively refers to both acrylic acid and methacrylic acid, and the term "(meth)acrylamide" inclusively refers to both acrylamide and methacrylamide.

The dental resin-reinforced glass ionomer cement kit of the present invention comprises a dental tooth surface treatment material composition (i) and a dental resin-reinforced glass ionomer cement composition (ii). The dental tooth surface treatment material composition contains (A) water-soluble organic solvent, (B) water and (C) polymerizable monomer having acid anhydride structure as essential components. Further, the dental resin-reinforced glass ionomer cement composition (ii) contains (D) acid-reactive glass powder, (B) water, (E) polymer of acidic group-containing polymerizable monomer, (F) tri or more functional (meth)acrylamide-based polymerizable monomer, (G) hydroxyl group-containing (meth)acrylate-based polymerizable monomer and (H) polymerization initiator as essential components.

The dental resin-reinforced glass ionomer cement kit of the present invention may consist of the dental tooth surface treatment material composition (i) and the dental resin-reinforced glass ionomer cement composition (ii).

In the dental resin-reinforced glass ionomer cement kit of the present invention, the (C) polymerizable monomer having acid anhydride structure may be a compound represented by formula (1): 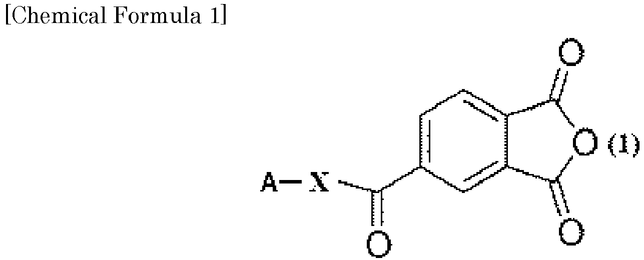 (In the formula, X represents a linear or branched alkylene group having 1 to 10 carbon atoms which may have an ether bond, an amide bond, an ester bond and/or a substituent, and A is (meth)acryloyloxy group or (meth)acrylamide group.)

In the dental resin-reinforced glass ionomer cement kit of the present invention, the (C) polymerizable monomer having acid anhydride structure may be at least one selected from the group consisting of 4-(meth)acryloyloxyethyl trimellitic anhydride, 4-(meth)acryloylaminoethyl trimethic anhydride, 4-(meth)acryloyloxyethyl trimellitic acid amide anhydride, 4-(meth)acryloylaminoethyltrimethic acid amide anhydride.

In the dental resin-reinforced glass ionomer cement kit of the present invention, the (A) water-soluble organic solvent may be at least one selected from the group consisting of ethanol, isopropanol and acetone.

In the dental resin-reinforced glass ionomer cement kit of the present invention, the (F) tri- or more functional (meth)acrylamide-based polymerizable monomer may be a compound represented by formula (2): 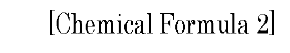 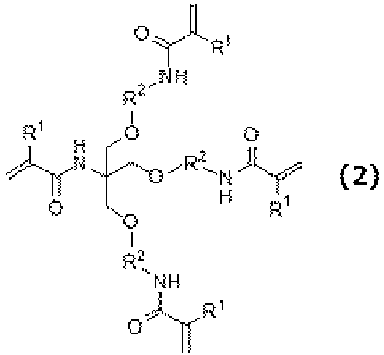 (In the formula, wherein R¹ represents a hydrogen atom or a methyl group and R¹s may be the same or different from each other, and R² represents a linear or branched alkylene group having 2 to 6 carbon atoms which may have a substituent and R²s may be the same or different from each other.)

In the dental resin-reinforced glass ionomer cement kit of the present invention, the dental resin-reinforced glass ionomer cement composition (ii) may further contain (I) acidic group-containing polymerizable monomer.

In the dental resin-reinforced glass ionomer cement kit of the present invention, the dental tooth surface treatment material composition (i) may further contain (J) 10-(meth)acryloyloxydecyl dihydrogen phosphate in the range of 0.1 % by mass or more and 10 % by mass or less.

In the dental resin-reinforced glass ionomer cement kit of the present invention, the dental resin-reinforced glass ionomer cement composition (ii) may contain (H) polymerization initiator in the range of 0.01% by mass or more and 10 % by mass or less.

In the dental resin-reinforced glass ionomer cement kit of the present invention, the dental resin-reinforced glass ionomer cement composition (ii) may contain (F) tri or more functional (meth)acrylamide-based polymerizable monomer in the range of 0.1% by mass or more and 30 % by mass or less.

First, each component compounded in the dental tooth surface treatment material composition (i) in the present invention is described in detail.

The dental tooth surface treatment material composition (i) in the present invention contains (A) water-soluble organic solvent for the purpose of dissolving (C) polymerizable monomer having acid anhydride structure. Specific examples of the (A) water-soluble organic solvent include acetone, ethanol, methanol, acetylacetone, methyl ethyl ketone, 1-propanol, isopropanol, 1-butanol, 1,4-dioxane, tetrahydrofuran, ethylene glycol, propylene glycol and phenol, but are not limited thereto. These (A) water-soluble organic solvents may be used alone or in combination of two or more thereof. Among these, ethanol, isopropanol or acetone can be used particularly from the viewpoint of drying.

The content of the (A) water-soluble organic solvent in the dental tooth surface treatment material composition (i) is within a range of 20 % by mass or more and 79 % by mass or less. When the content of the (A) water-soluble organic solvent is less than 20 % by mass or exceeds 79 % by mass, sufficient adhesive strength cannot be obtained.

The dental tooth surface treatment material composition (i) in the present invention contains (B) water for the purpose of permeating (C) polymerizable monomer having acid anhydride structure into the tooth substance. Any water can be is used as the (B) water as long as it does not contain impurities adversely affecting on the adhesive property of the dental tooth surface treatment material composition (i) to the tooth substance and the curability and mechanical characteristic of the dental resin-reinforced glass ionomer cement composition (ii). Specifically, distilled water, purified water and ion-exchanged water can be used. The content of the (B) water in the dental tooth surface treatment material composition (i) is within a range of 20 % by mass or more and 79 % by mass or less. When the content of the (B) water is less than 20 % by mass or exceeds 79 % by mass, sufficient adhesive strength cannot be obtained.

The dental tooth surface treatment material composition (i) in the present invention contains (C) polymerizable monomer having acid anhydride structure for the purpose of modifying a hydrophilic tooth surface to be somewhat hydrophobic to improve affinity with various polymerization monomers contained in the dental resin-reinforced glass ionomer cement composition (ii). Specific examples of the acid anhydride structure include a succinic anhydride structure, a maleic anhydride structure, a phthalic anhydride structure and the like, but are not limited thereto. Moreover, specific examples of polymerizable group include a (meth)acryloyloxy group, a (meth)acrylamide group and the like, but are not limited thereto. The number of polymerizable groups is also not particularly limited, but it can be monofunctional because it easily penetrates into tooth substance. There is no problem even if the hydrocarbon bonded to the polymerizable group has a hydroxyl group, a halogen atom, an amino group, a glycidyl group, an ether bond, an amide bond, an ester bond or the like.

Among them, the (C) polymerizable monomer having acid anhydride structure may be a compound represented by formula (1). 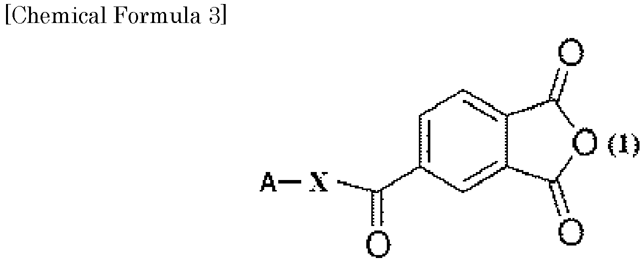 (In the formula, X represents a linear or branched alkylene group having 1 to 10 carbon atoms which may have an ether bond, an amide bond, an ester bond and/or a substituent, and A is (meth)acryloyloxy group or (meth)acrylamide group.)

The (C) polymerizable monomer having acid anhydride structure may be at least one selected from the group consisting of 4-(meth)acryloyloxyethyl trimellitic anhydride, 4-(meth)acryloylaminoethyl trimethic anhydride, 4-(meth)acryloyloxyethyl trimellitic acid amide anhydride, 4-(meth)acryloylaminoethyltrimethic acid amide anhydride.

The content of the (C) polymerizable monomer having acid anhydride structure in the dental tooth surface treatment material composition (i) is within a range of 1 % by mass or more and 30 % by mass or less. When the content of the (C) polymerizable monomer having acid anhydride structure is less than 1 % by mass, sufficient adhesive strength cannot be obtained. When the content of the (C) polymerizable monomer having acid anhydride structure exceeds 30 % by mass, application property deteriorates.

The dental tooth surface treatment composition (i) in the present invention may contain (J) 10-(meth)acryloyloxydecyl dihydrogen phosphate which is an acidic group-containing polymerizable monomer, for the purpose of further improving the adhesive property to tooth substance. Furthermore, the (J) 10-(meth)acryloyloxydecyl dihydrogen phosphate has a feature that it does not easily reduce storage stability even if the (J) 10-(meth)acryloyloxydecyl dihydrogen phosphate is additionally compounded into the dental tooth surface treatment material composition (i) in the present invention, unlike other acidic group-containing polymerizable monomers. The content of the (J) 10-(meth)acryloyloxydecyl dihydrogen phosphate in the dental tooth surface treatment material composition (i) may be within a range of 0.1 % by mass or more and 10 % by mass or less. When the content of the (J) 10-(meth)acryloyloxydecyl dihydrogen phosphate is less than 0.1 % by mass, there is a case that sufficient adhesive strength cannot be obtained. When the content of the (J) 10-(meth)acryloyloxydecyl dihydrogen phosphate exceeds 10 % by mass, there is a case that application property deteriorates.

The dental tooth surface treatment material composition (i) in the present invention may optionally contain an acidic group-containing polymerizable monomer other than (J) 10-(meth)acryloyloxydecyl dihydrogen phosphate for the purpose of balancing the adhesive property to enamel and the adhesive property to dentin as long as the storage stability is not reduced. Specifically, the same acidic group-containing polymerizable monomer as the (I) acidic group-containing polymerizable monomer that can be compounded in the dental resin-reinforced glass ionomer cement composition (ii) described below can be used. However, the amount thereof may be 1/3 or less, or 1/5 or less, or 1/10 or less of the (C) polymerizable monomer having acid anhydride structure contained in the dental tooth surface treatment material composition (i). The dental tooth surface treatment material composition (i) in the present invention may contain no acidic group-containing polymerizable monomer other than (J) 10-(meth)acryloyloxydecyl dihydrogen phosphate.

The dental tooth surface treatment material composition (i) in the present invention may contain a polymerization initiator for the purpose of improving polymerization activity at the adhesive interface with the dental resin-reinforced glass ionomer cement composition (ii) as long as the storage stability is not reduced. Specifically, the same polymerization initiator as the (H) polymerization initiator that can be compounded in the dental resin-reinforced glass ionomer cement composition (ii) described below can be used. The content of the (H) polymerization initiator is not particularly limited, but is generally within a range of 0.01 % by mass or more and 10 % by mass or less in dental tooth surface treatment material composition (i). When the content of the (H) polymerization initiator is less than 0.01 % by mass, there is a case that the polymerization activity is not improved. When the content of the (H) polymerization initiator exceeds 10 % by mass, there is a case that there is a case that sufficient adhesive strength cannot be obtained.

The dental tooth surface treatment material composition (i) in the present invention may optionally contain a polymerizable monomer having no acid anhydride structure or no acid group. Examples of such polymerizable monomers include Examples of such polymerizable monomers include various polymerizable monomers that can be compounded in the dental resin-reinforced glass ionomer cement composition (ii) described below. However, as the compounding amount of such a polymerizable monomer increases, there is a case that it becomes difficult to dry or stickiness tends to occur after drying after applying the dental tooth surface treatment material composition (i) to the tooth surface. As a result, slippage may occur with the dental resin-reinforced glass ionomer cement composition (ii) to be applied next, and therefore there is a case that it is particularly difficult to perform the filling operation. Thus, the total compounding amount of the a polymerizable monomer having no acid anhydride structure or no acid group may be 10 % by mass or less, 5 % by mass or less in the dental tooth surface treatment material composition (i), and no polymerizable monomer having no acid anhydride structure or no acid group may be compounded.

Next, each component compounded in the dental resin-reinforced glass ionomer cement composition (ii) in the present invention is described in detail. The dental resin-reinforced glass ionomer cement composition (ii) can be provided in various forms such as a powder-liquid type and a two-paste type.

For example, the powder-liquid type dental resin-reinforced glass ionomer cement composition (ii) consists of a powder material containing the (D) acid-reactive glass powder, and a liquid material containing the (B) water, the (E) polymer of acidic group-containing polymerizable monomer, the (F) tri or more functional (meth)acrylamide-based polymerizable monomer and the (G) hydroxyl group-containing (meth)acrylate-based polymerizable monomer, and contains the (H) polymerization initiator in at least one of the powder material and the liquid material.

For example, in the case of the two-paste type dental resin-reinforced glass ionomer cement composition (ii), at least one of the two pastes may contain the (D) acid-reactive glass powder, the (E) polymer of acidic group-containing polymerizable monomer, the (B) water, the (G) hydroxyl group-containing (meth)acrylate-based polymerizable monomer, the (F) tri or more functional (meth)acrylamide-based polymerizable monomer, and the (H) polymerization initiator. In this case, the paste containing the (B) water may not contain at least one of the (D) acid-reactive glass powder and the (E) polymer of acidic group-containing polymerizable monomer. Further, at least one of the two pastes further may contain the (I) acidic group-containing polymerizable monomer, and the paste containing the (B) water may not contain at least one of the (D) acid-reactive glass powder and the (I) acidic group-containing polymerizable monomer.

The (D) acid-reactive glass powder that can be used in the dental resin-reinforced glass ionomer cement composition (ii) in the present invention needs to contain an acid-reactive element and fluorine. Because the (D) acid-reactive glass powder includes an acid reactive element, the acid-base reaction of the (D) acid-reactive glass powder with the acid group contained in the (E) polymer of acidic group-containing polymerizable monomer described later progresses in the presence of (B) water. Specific examples of an acid reactive element include sodium, potassium, calcium, strontium, barium, lanthanum, aluminum and zinc, but are not limited thereto. One or two or more kinds of these acid reactive element may be contained and a content thereof is not particularly limited.

Further, it is preferable that the (D) acid reactive glass powder includes an X-ray impermeable element in order to impart X-ray contrast property to the dental resin-reinforced glass ionomer cement composition (ii) in the present invention. Specific examples of an X-ray impermeable element include strontium, lanthanum, zirconium, titanium, yttrium, ytterbium, tantalum, tin, tellurium, tungsten and bismuth, but are not limited thereto. In addition, other element contained in the (D) acid reactive glass powder is not particularly limited and the (D) acid reactive glass powder in the present invention may include various elements.

Specific examples of the (D) acid-reactive glass powder include aluminosilicate glass, borosilicate glass, aluminoborate glass, boro aluminosilicate glass, phosphate glass, borate glass, silica glass wherein the above described acid reactive element, fluorine element and X-ray impermeable element are contained, but are not limited thereto.

Further, a particle shape of the (D) acid-reactive glass powder is not particularly limited, but arbitral particle shapes such as spherical, needle-like, plate-like, ground-like, and scaly-shape may be used without any limitation. These (D) acid-reactive glass powder may be used alone or in combination of two or more thereof.

A preparing process of the (D) acid-reactive glass powder is not particularly limited, and an acid reactive glass powder prepared by any process such as a melting process, a vapor phase process and a sol-gel process may be used without any problem. Among them, the (D) acid-reactive glass powder prepared by a melting method or a sol-gel method which can easily control a kind of element contained in the (D) acid-reactive glass powder and the content thereof can be used.

As the (D) acid-reactive glass powder, fillers which are generally sold may be used without processing such as grinding, but it is possible to adjust to a desired average particle diameter by grinding appropriately according to the application or purpose and usage of the dental resin-reinforced glass ionomer cement composition (ii) in the present invention. A grinding method is not particularly limited, but an acid-reactive glass powder obtained by grinding which use any of wet or dry grinding methods may be used. Specifically, the acid reactive glass powder may be ground by a high speed rotating mill such as a hammer mill and a turbo-mill, a container driving medium mill such as a ball mill and a vibration mill, a medium stirring mill such as a sand grinder and attritor, and a jet mill and the like.

For example, when the dental resin-reinforced glass ionomer cement composition (ii) in the present invention is used as a material for filling or abutment building, because high mechanical strength is required, the (D) acid-reactive glass powder may have an average particle size in the range of 0.01 to 30.0 um, and in the range of 0.01 to 10.0 pm.

In addition, when the dental resin-reinforced glass ionomer cement composition (ii) in the present invention is used as a material for luting, because a thin film thickness is required, the (D) acid-reactive glass powder may have an average particle size in the range of 0.01 to 10.0um, more preferably in the range of 0.01 to 5.0 pm.

When the average particle diameter of the (D) acid-reactive glass powder is less than 0.01 um, the surface area of the acid-reactive glass powder increases and it becomes impossible to contain the acid-reactive glass powder in a large amount into the composition, therefore there is a risk that deterioration in mechanical characteristics may be caused. In addition, there is a case that the viscosity of the mixture may increase and the operability may deteriorate.

In the case of using as a material for filling or abutment building, when the average particle diameter of the (D) acid-reactive glass powder exceeds 30.0 um, the surface of the material after polishing becomes rough, therefore coloring may be caused. Further, in case of using as a material for luting, when the average particle diameter of the (D) acid-reactive glass powder exceeds 10.0 um, because the film thickness becomes thick, the attached dental prosthesis device is lifted and therefore there is a risk that the intended fit cannot be obtained.

In order to adjust operability, hardening characteristics, mechanical characteristics and the like of the dental resin-reinforced glass ionomer cement composition (ii) in the present invention, the (D) acid-reactive glass powder may be treated with various surface treatments, heat treatment, aggregating treatment in a liquid phase or a vapor phase, microcapsulation in which surface is enclosed with an organic substance, grafting in which a surface is functionalized with an organic substance and the like to such a range that the acid-base reaction of the acid reactive glass powder with the acid group contained in the (E) polymer of acidic group-containing polymerizable monomer described later is not influenced. These treatments can be performed alone or in a combination of a few kinds, with no problems. Among them, the surface treatment and heat treatment can be performed because it is easy to control various characteristics and those are superior in productivity.

Specific examples of the surface treating method of the (D) acid-reactive glass powder include washing with acid such as phosphoric acid or acetic acid, surface treatment with acidity compound such as tartaric acid or polycarboxylic acid, surface treatment with fluoride such as aluminum fluoride and surface treatment with silane compound such as γ-methacryloyloxypropyltrimethoxysilane or tetramethoxy silane. The surface treating method which can be used in the present invention is not limited the above described method and these surface treating methods can be used alone or in a combination thereof.

Specific examples of the heat treating method of the (D) acid-reactive glass powder include a treating method which includes heating for a range of 1 hour to 72 hours within a such range of 100 °C or more to 800 °C or less using electric furnace. The heat treating method which can be used in the present invention is not limited the above described method and any method of uni-temperature and multi-stage temperature can be used as the heat treating method without any problem.

The (B) water that can be used in the dental resin-reinforced glass ionomer cement composition (ii) in the present invention is as described in the dental tooth surface treatment material composition (i). In the two-paste type dental resin-reinforced glass ionomer cement composition (ii), a paste containing (B) water may contain (E) polymer of acidic group-containing polymerizable monomer.

Any polymer can be used as the (E) polymer of acidic group-containing polymerizable monomer which is used in the dental resin-reinforced glass ionomer cement composition (ii) in the present invention as long as it is a polymer in which a polymerizable monomer having at least one or more an acid group in a molecule is without any limitation. In the case of the powder-liquid type dental resin-reinforced glass ionomer cement composition (ii), the (E) polymer of acidic group-containing emulsion monomer may be compounded in a liquid material, but there is no problem even if a part thereof is compounded in a powder material.

As the acidic group-containing polymerizable monomers which may be used for obtaining the (E) polymer of acidic group-containing polymerizable monomer, any acidic group-containing polymerizable monomers may be used regardless of the type of acidic group. In addition, any acidic group-containing polymerizable monomers may be used regardless of the number or the type of radical polymerizable unsaturated groups (monofunctional groups or multifunctional) of the acidic group-containing polymerizable monomer.

Specific examples of the acidic group of the acidic group-containing polymerizable monomer are not limited to, but include a phosphate group, a pyrophosphate group, a phosphonyl group, a carboxyl group, a sulfonyl group, and a thiophosphate group, but are not limited to. In the dental resin-reinforced glass ionomer cement composition (ii) in the present invention, it is possible that other acidic group-containing polymerizable monomer other than the acidic group-containing polymerizable monomer including a phosphate group, a pyrophosphate group, a phosphonyl group, a carboxyl group, a sulfonyl group and/or a thiophosphate group is not contained.

Specific examples of the unsaturated polymerizable group of the acidic group-containing polymerizable monomer are not limited to, but include a (meth) acryloyloxy group, a (meth) acrylamide group, a styryl group, a vinyl group, and an aryl group. Among these unsaturated groups, it is possible to be a (meth) acryloyloxy group and a (meth) acrylamide group, and to be a (meth) acryloyloxy group.

Further, these acidic group-containing polymerizable monomers may contain together other functional group such as an alkyl group, halogen, an amino group, a glycidyl group and a hydroxy group in a molecule.

Specific examples of an acidic group-containing polymerizable monomer which may be used for obtaining the (E) polymer of acidic group-containing polymerizable monomer and has a (meth)acryloyloxy group as an unsaturated group, are specifically listed below.

Specific examples of an acidic group-containing polymerizable monomer which has a phosphate group are not limited to, but include (meth)acryloyloxymethyl dihydrogen phosphate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, bis [2-(meth) acryloyloxyethyl] hydrogensphosphate, bis [3-(meth) acryloyloxypropyl] hydrogen phosphate, bis [4-(meth) acryloyloxybutyl] hydrogen phosphate, bis [6-(meta) acryloyloxyhexyl] hydrogen phosphate, bis [8-(meth) acryloyloxyoctyl] hydrogen phosphate, bis [9-(meth) acryloyloxynonyl] hydrogen phosphate, bis [10-(meth) acryloyloxydecyl] hydrogen phosphate, 1,3-di(meth) acryloyloxypropyl-2-dihydrogenphosphate, 2-(meth) acryloyloxyethylphenyl hydrogen phosphate, and 2-(meth) acryloyloxyethyl 2'-bromoethyl hydrogen phosphate.

Specific examples of an acidic group-containing polymerizable monomer which has a pyrophosphate group are not limited to, but include, bis [2-(meth) acryloyloxyethyl] pyrophosphate, bis [3-(meth) acryloyloxypropyl] pyrophosphate, bis [4-(meth) acryloyloxybutyl] pyrophosphate, bis [5-(meth) acryloyloxypentyl] pyrophosphate, bis [6-(meth) acryloyloxyhexyl] pyrophosphate, bis [7-(meth) acryloyloxyheptyl] pyrophosphate, bis [8-(meth) acryloyloxyoctyl] pyrophosphate, bis [9-(meth) acryloyloxynonyl] pyrophosphate, bis [10-(meth) acryloyloxydecyl] pyrophosphate, bis [12-(meth) acryloyloxydodecyl] pyrophosphate, tris [2-(meth) acryloyloxyethyl] pyrophosphate and tetra [2-(meth) acryloyloxyethyl] pyrophosphate.

Specific examples of an acidic group-containing polymerizable monomer which has a phosphonyl group are not limited to, but include 5-(meth) acryloyloxypentyl-3-phosphonopropionate, 6-(meth) acryloyloxyhexyl-3-phosphonopropionate, 10-(meth) acryloyloxydecyl-3-phosphonopropionate, 6-(meth) acryloyloxyhexyl-3-phosphonoacetate, 10-(meth) acryloyloxydecyl-3-phosphonoacetate, and (meth) acryloyloxyethl phenyphosphonoacetate.

Specific examples of an acidic group-containing polymerizable monomer which has a carboxyl group are not limited to, but include (meth) acrylic acid, 2-chloro acrylic acid, 3-chloro(meth)acrylic acid, 2-cyano acrylic acid, aconitic acid, mesaconic acid, maleic acid, maleic anhydride, itaconic acid, itaconic anhydride, fumaric acid, glutaconic acid, citraconic acid, utraconic acid, 1,4-di(meth) acryloyloxyethylpyromellitic acid, 6-(meth) acryloyloxynaphthalene-1,2,6-tricarboxylic acid, 1-buten-1,2,4-tricarboxylic acid, 3-buten-1,2,3-tricarboxylic acid, N-(meth) acryloyl-p-aminobenzoic acid, N-(meth) acryloyl-5-aminosalicylic acid, 4-(meth) acryloyloxyethyltrimellitic acid and anhydride thereof, 4-(meth) acryloyloxybutyltrimellitic acid and anhydride thereof, 2-(meth) acryloyloxybenzoic acid, β-(meth) acryloyloxyethyl hydrogen succinate, β-(meth) acryloyloxyethyl hydrogen maleate, 11-(meth) acryloyloxy-1,1-undecanedicarboxylic acid, p-vinylbenzoic acid, 4-(meth) acryloyloxyethoxycarbonylphthalic acid, 4-(meth) acryloyloxybutyloxycarbonylphthalic acid, 4-(meth) acryloyloxyhexyloxycarbonylphthalic acid, 4-(meth) acryloyloxyoctyloxycarbonylphthalic acid, 4-(meth) acryloyloxydecyloxycarbonylphthalic acid and anhydride thereof, 5-(meth) acryloylaminopentylcarboxylic acid, 6-(meth) acryloyloxy-1,1-hexanedicarboxylic acid, 8-(meth) acryloyloxy-1,1-octanedicarboxylic acid, 10-(meth) acryloyloxy-1,1-decanedicarboxylic acid, and 11-(meth) acryloyloxy-1,1-undecanedicarboxylic acid.

Specific examples of an acidic group-containing polymerizable monomer which has a sulfonyl group are not limited to, but include 2-(meth) acrylamido-2-methylpropanesulfonic acid, styrene sulfonic acid, 2-sulfoethyl (meth) acrylate, 4-(meth) acryloyloxy benzenesulfonic acid, and 3-(meth) acryloyloxy propanesulfonic acid.

Specific examples of an acidic group-containing polymerizable monomer which has a thiophosphate group are not limited to, but include, 2-(meth) acryloyloxyethyl dihydrogenthiophosphate, 3-(meth) acryloyloxypropyl dihydrogenthiophosphate, 4-(meth) acryloyloxybutyl dihydrogenthiophosphate, 5-(meth) acryloyloxypentyl dihydrogenthiophosphate, 6-(meth) acryloyloxyhexyl dihydrogenthiophosphate, 7-(meth) acryloyloxyheptyl dihydrogenthiophosphate, 8-(meth) acryloyloxyoctyl dihydrogenthiophosphate, 9-(meth) acryloyloxynonyl dihydrogenthiophosphate, 10-(meth) acryloyloxydecyl dihydrogenthiophosphate.

These acidic group-containing polymerizable monomers can be used not only singly but also in combinations of a plurality thereof for synthesizing the (E) polymer of the acidic group-containing polymerizable monomer, without any problem. In addition, the (E) polymer of the acidic group-containing polymerizable monomer may be synthesized by copolymerizing an acidic group-containing polymerizable monomer containing one or more acidic group in a molecule and a polymerizable monomer containing no acidic group, without any problem.

It is possible to use an α,β-unsaturated carboxylic acid based acidic group-containing polymerizable monomers among these acidic group-containing polymerizable monomers. The α,β-unsaturated carboxylic acid based acidic group-containing polymerizable monomer which can be used in this case is not particularly limited and may be used regardless of the number of carboxy groups in the molecule or the existence of a carboxylic anhydride group or other substituents. In the dental resin-reinforced glass ionomer cement composition in the present invention, a polymer of acidic group-containing polymerizable monomer other than α,β-unsaturated carboxylic acid based acidic group-containing polymerizable monomer may be not contained.

Specific examples of an α,β-unsaturated carboxylic acid based acidic group-containing polymerizable monomer are not limited to, but include (meth) acrylic acid, 2-chloro acrylic acid, 3-chloro (meth) acrylic acid, 2-cyano acrylic acid, aconitic acid, mesaconic acid, maleic acid, maleic anhydride, itaconic acid, itaconic anhydride, fumaric acid, glutaconic acid, citraconic acid, utraconic acid, tiglic acid, 1-buten-1,2,4-tricarboxylic acid, and 3-buten-1,2,3-tricarboxylic acid.

The method of polymerizing various polymerizable monomers is not particularly limited, and a polymer polymerized by any methods such as solution polymerization, suspension polymerization, emulsion polymerization or the like, may be used without any limitation. In addition, a polymerization initiator and a chain transfer agent which can be used at the time of synthesis of a polymer may be appropriately selected in order to obtain a desired polymer. The (E) polymer of polymer of acidic group-containing polymerizable monomer obtained by such way can be used alone, or in a combination of a few kinds.

The obtained (E) polymer of the acidic group-containing polymerizable monomer may be used after neutralization reaction of a part of the acidic group thereof by using an alkali metal hydroxide such as sodium hydroxide, potassium hydroxide and lithium hydroxide, an alkali metal carbonate such as sodium carbonate, potassium carbonate and lithium carbonate, and a bicarbonate of an alkali metal such as sodium hydrogen carbonate, potassium hydrogen carbonate, lithium hydrogen carbonate or the like, for the purpose of adjusting the working time and hardening time, or for the purpose of improving storage stability. The compound used for this neutralization is not limited to these, and there is no problem even if the compound may be used alone or in combination of several kinds.

Furthermore, there is no problem even if the (E) polymer of acidic group-containing polymerizable monomer has a radical polymerizable unsaturated group. However, the (E) polymer of acidic group-containing polymerizable monomer having an unsaturated group has a relatively low solubility in (B) water, and there is a tendency that its compounding amount is low, therefore, there is a case that the mechanical property of the hardened product may be deteriorated. Thus, it is possible that the the (E) polymer of acidic group-containing polymerizable monomer does not have an unsaturated group.

Among them, it is possible to use the (E) polymer of acidic group-containing polymerizable monomer synthesized from only acrylic acid as a starting material (poly acrylic acid) or the (E) polymer of an acid group-containing polymerizable monomer (copolymer) synthesized from two or more kinds of starting materials such as acrylic acid and maleic acid, acrylic acid and maleic anhydride, acrylic acid and itaconic acid, and acrylic acid and 3- butene-1,2,3-tricarboxylic acid.

A weight average molecular weight of the polymer of (E) acid group-containing polymerizable monomer is not limited particular, but may be in a rage of 10,000 to 500,000, may be in a rage of 20,000 to 300,000 and may be in a rage of 20,000 to 200,000. When a weight average molecular weight of the (E) polymer of acidic group-containing polymerizable monomer is less than 10,000, the mechanical characteristic of a hardened product may tend to decrease too much to cause problem in durability of the hardened product. On the other hand, when a weight average molecular weight of the (E) acid group-containing polymerizable monomer increases above 500,00, viscosity of the mixture in mixing the dental resin-reinforced glass ionomer cement composition (ii) becomes high and there may be a problem in operability.

As the (F) tri or more functional (meth)acrylamide-based polymerizable monomer which can be used in the dental resin-reinforced glass ionomer cement composition (ii) in the present invention, any polymerizable monomer as long as the polymerizable monomer contains three or more (meth)acrylamide groups in the molecule can be used without any limitation. In the case that the (F) tri or more functional (meth)acrylamide-based polymerizable monomer does not have an acidic group and/or a hydroxyl group, the effect in improving the surface curability and the coloring resistance and reducing the water absorption expansion is easily obtained.

Specific examples of the (F) tri or more functional (meth)acrylamide-based polymerizable monomer include those represented by following formula (2) and formula (3). In the dental resin-reinforced glass ionomer cement composition (ii) in the present invention, it is possible that tri or more functional (meth)acrylamide-based polymerizable monomer other than the tri or more functional (meth)acrylamide-based polymerizable monomer represented by following formula (2) and formula (3) is not contained.

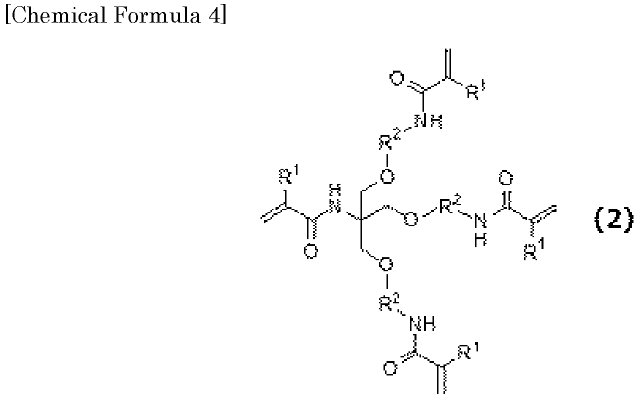

(In formula, R¹ represents a hydrogen atom or a methyl group and R¹s may be the same or different from each other, and R² represents a linear or branched alkylene group having 2 to 6 carbon atoms which may have a substituent and R²s may be the same or different from each other.)

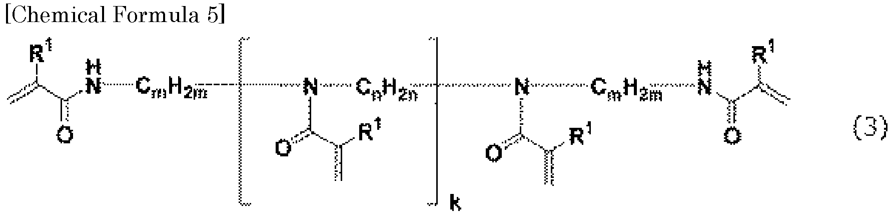

(In formula, R¹ represents a hydrogen atom or a methyl group. The "m" represents an integer of 2 to 4. The "n" represents an integer of 2 to 4. The "k" represents 0 or 1. A plurality of R¹s and the "b"s may be the same or different from each other.)

More specific examples of the (F) tri or more functional (meth)acrylamide-based polymerizable monomer include those represented by following formula (4) and formulas (5) to (8).

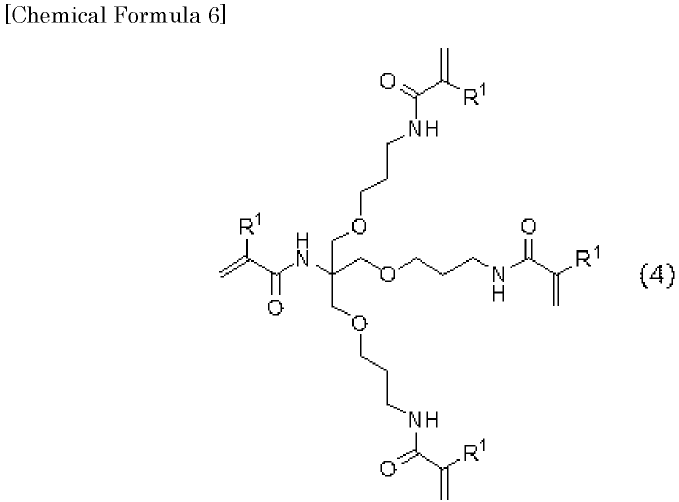

(In formula, R¹ represents a hydrogen atom or a methyl group and R¹s may be the same or different from each other.)

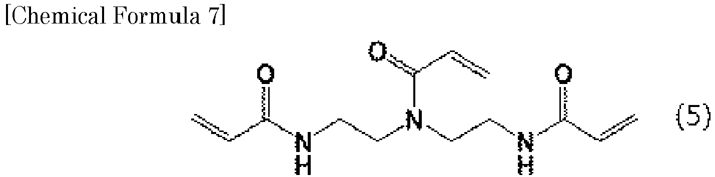

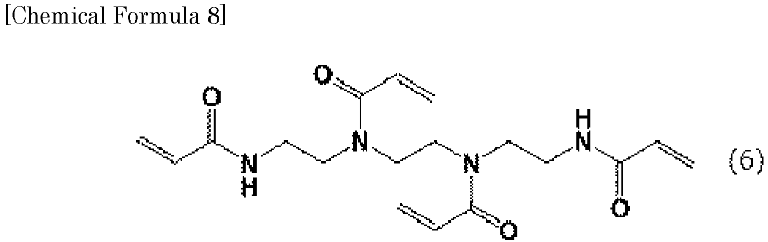

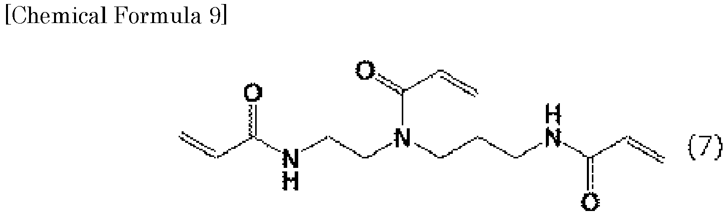

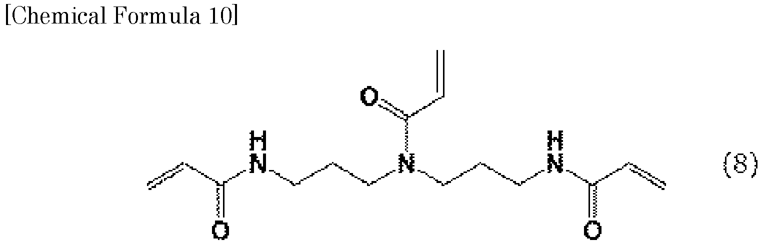

Among these, it is possible to be a tetra or more functional (meth)acrylamide-based polymerizable monomer, to be the polymerizable monomer represented by the above formula (2), and to be the polymerizable monomer represented by the above formula (4), and to be the polymerizable monomer represented by the above formula (4) in which all R¹s are hydrogen atoms in the formula. In the dental resin-reinforced glass ionomer cement composition (ii) in the present invention, it is possible that tri or more functional (meth)acrylamide-based polymerizable monomer other than tetra or more functional (meth)acrylamide-based polymerizable monomer is not contained. In the dental resin-reinforced glass ionomer cement composition (ii) in the present invention, it is possible that tri or more functional (meth)acrylamide-based polymerizable monomer other than polymerizable monomer by the above formula (2) is not contained. In the dental resin-reinforced glass ionomer cement composition (ii) in the present invention, it is possible that tri or more functional (meth)acrylamide-based polymerizable monomer other than polymerizable monomer by the above formula (4) is not contained. In the dental resin-reinforced glass ionomer cement composition (ii) in the present invention, it is possible that tri or more functional (meth)acrylamide-based polymerizable monomer other than polymerizable monomer by the above formula (4) in which all R¹s are hydrogen atoms in the formula is not contained.

Any polymerizable monomer can be used as the (G) hydroxyl group-containing (meth)acrylate-based polymerizable monomer which can be used as the dental resin-reinforced glass ionomer cement composition (ii) in the present invention as long as the monomer has at least one or more hydroxyl group in a molecule and at least one or more (meth)acrylate group as a radical polymerizable unsaturated group, without any limitation.

Specific examples of the (G) hydroxyl group-containing (meth)acrylate-based polymerizable monomer include a monofunctional (meth)acrylate-based polymerizable monomer such as 2-hydroxyethyl (meth)acrylate (2-HEMA), 2-hydroxypropyl (meth)acrylate, 3-hydroxypropyl (meth)acrylate, 4-hydroxybutyl (meth)acrylate, 5-hydroxypentyl (meth)acrylate, 6-hydroxyhexyl (meth)acrylate, 10-hydroxydecyl (meth)acrylate, diethylene glycol mono(meth)acrylate, triethylene glycol mono(meth)acrylate, tetraethylene glycol mono(meth)acrylate, polyethylene glycol mono(meth)acrylate, dipropylene glycol mono(meth)acrylate, polypropylene glycol mono(meth)acrylate, 2,3-dihydroxypropyl (meth)acrylate, glycerin mono(meth)acrylate, erythritol mono(meth)acrylate, and an addition product of phenols and glycidyl (meth)acrylate, for example, 2-hydroxy-3-phenoxypropyl (meth)acrylate and 2-hydroxy-3-naphthoxypropyl (meth)acrylate, and a polyfunctional (meth)acrylate-based polymerizable monomers such as 2-hydroxypropyl-1,3-di(meth)acrylate (GDMA), 3-hydroxypropyl-1,2-di(meth)acrylate, bisphenol A diglycidyl (meth)acrylate (Bis-GMA), and 2-hydroxy-3-acryloyloxypropyl methacrylate (GDA). Further, a polyfunctional (meth)acrylate-based polymerizable monomer in which two of more hydroxyl groups of sugar alcohols (erythritol, arabinitol, xylitol, ribitol, iditol, galactitol, sorbitol, mannitol, etc.), monosaccharides (arabinose, xylose, mannose, galactose, fructose, etc.), disaccharides (sucrose, maltose, lactose, trehalose, etc.) and a trisaccharide (maltotriose, raffinose, etc.) are substituted with a substituent having a polymerizable unsaturated group can also be preferably used, but specific examples are not limited thereto.

Among them, 2-hydroxyethyl (meth)acrylate, 2-hydroxypropyl (meth)acrylate, 2,3-dihydroxypropyl (meth)acrylate, bisphenol A diglycidyl (meth)acrylate (Bis-GMA), 2-hydroxypropyl-1,3-di(meth)acrylate (GDMA), and 2-hydroxy-3-acryloyloxypropyl methacrylate (GDA) are particularly preferred. Two or more kinds of these (G) hydroxyl group-containing (meth)acrylate-based polymerizable monomers may be appropriately used in combination, if desired. In the dental resin-reinforced glass ionomer cement composition (ii) in the present invention, it is possible that hydroxyl group-containing (meth)acrylate-based polymerizable monomer other than 2-hydroxypropyl (meth)acrylate, 2,3-dihydroxypropyl (meth)acrylate, bisphenol A diglycidyl (meth)acrylate (Bis-GMA), 2-hydroxypropyl-1,3-di(meth)acrylate (GDMA), and 2-hydroxy-3-acryloyloxypropyl methacrylate (GDA) is not contained.

In the dental resin-reinforced glass ionomer cement composition (ii) in the present invention, it is preferable that the (G) hydroxyl group-containing (meth)acrylate-based polymerizable monomer contains both a mono functional (meth)acrylate-based polymerization monomer that has a hydroxyl group and a bi to tetra functional (meth)acrylate-based polymerizable monomer that has a hydroxyl group and the compounding ratio of the mono functional (meth)acrylate-based polymerization monomer that has a hydroxyl group and the bi to tetra functional (meth)acrylate-based polymerizable monomer that has a hydroxyl group is 1:2 to 4:1 by mass. By using such a combination and compounding ratio of the (G) hydroxyl group-containing (meth)acrylate-based polymerizable monomer, since the (B) water, the (E) polymer of acidic group-containing polymerizable monomer, and the (F) tri or more functional (meth)acrylamide-based polymerizable monomer are easily and uniformly compatible, the mechanical properties and transparency after hardening can be improved. Furthermore, it is more preferable that the bi to tetra functional (meth)acrylate-based polymerizable monomer that has a hydroxyl group is a bifunctional (meth)acrylate-based polymerizable monomer having a hydroxyl group.

Any known photopolymerization initiators and/or chemical polymerization initiators can be used as the (H) polymerization initiator that can be used in the dental tooth surface treatment material composition (i) and the dental resin-reinforced glass ionomer cement composition (ii) in the present invention, without any limitation. In the case that the dental resin-reinforced glass ionomer cement composition (ii) is powder-liquid type, the (H) polymerization initiator may be incorporated in at least one of a powder material and a liquid material as long as the resin component can be sufficiently cured, and various polymerization initiator systems can be used.

Examples of the photopolymerization initiator include photosensitizers and photosensitizer/photopolymerization promotor or the like. Specific examples of thephotosensitizer may include α-diketones such as benzil, camphorquinone, α-naphtil, acetonaphtone, p,p'-dimethoxybenzil, p,p'-dichlorobenzylacetyl, pentadione, 1,2-phenanthrenquinone, 1,4-phenanthrenquinone, 3,4-phenanthrenquinone, 9,10-phenanthrenquinone and naphthoquinone; benzoin alkyl ethers such as benzoin, benzoin methyl ether and benzoin ethyl ether; thioxanthones such as thioxanthone, 2-chlorothioxanthone, 2-methylthioxanthone, 2-isopropylthioxanthone, 2-methoxythioxanthone, 2-hydroxythioxanthone, 2,4-diethylthioxanthone and 2,4-diisopropylthioxanthone; benzophenones such as benzophenone, p-chlorobenzophenone and p-methoxybenzophenone; acylphosphineoxides such as 2,4,6-trimethylbenzoyl diphenylphosphineoxide, bis(2,4,6-trimethylbenzoyl) phenylphosphine oxide, 2,6-dimethoxybenzoyl diphenylphosphine oxide and bis(2,6-dimethoxybenzoyl)phenylphosphine oxide; α-aminoacetophenones such as 2-benzyl-dimethylamino-1-(4-morpholinophenyl)-butanone-1, 2-benzyl-diethyl-amino-1-(4-morpholinophenyl)propanone-1; ketals such as benzyldimethylketal, benzyldiethylketal and benzyl(2-methoxyethylketal); coumarins such as 3-(4-methoxybenzoyl)coumarin, 3-benzoyl-5,7-dimethoxycoumarin, 3,3'-carbonyl bis(7-diethylaminocoumarin) and 3,3'-carbonyl bis(7-dibutylaminocoumarin), and titanocenes such as bis(cyclopentadienyl)-bis [2, 6-difluoro-3-(1-pyrolyl)phenyl] titanium, bis(cyclopentadienyl)-bis(pentanefluorophenyl)titanium and bis(cyclopentadienyl)-bis(2,3,5,6-tetrafluoro-4-disiloxyphenyl)-titanium, but are not limited to.

Specific examples of the photopolymerization promotor may include tetriary amines such as N,N-dimethylaniline, N,N-diethylaniline, N,N-di-n-butylaniline, N,N-dibenzylaniline, N,N-dimethyl-p-toluidine, N,N-dimethyl-m-Toluidine, N,N-diethyl-p-toluidine, p-bromo-N,N-dimethylaniline, m-chloro-N,N-dimethylaniline, p-dimethylaminobenzaldehyde, p-dimethylaminoacetophenone, p-dimethyl aminobenzoic acid, ethyl p-dimethylaminobenzoate, isoamyl p-dimethylaminobenzoate, N,N-dimethylanthranilic acid methyl ester, N,N-dihydroxyethylaniline, N,N-dihydroxyethyl-p-toluidine, p-dimethylaminophenyl alcohol, p-dimethylaminostyrene, N,N-dimethyl-3,5-xylidine, 4-dimethylaminopyridine, N,N-dimethyl-α-naphthylamine, N,N-dimethyl-β- naphthylamine, triethanolamine, tributylamine, tripropylamine, triethylamine, N-methyldiethanolamine, N-ethyldiethanolamine, N,N-dimethylhexylamine, N,N-dimethyldodecylamine, N,N-dimethylstearylamine, N,N-dimethylaminoethylmethacrylate, N, N-diethylaminoethylmethacrylate, 2,2' - (n-butyl imino)diethanol and other tertiary amines; secondary amines such as N-phenylglycine; barbiturates such as 5-butylbarbituric acid, 1-benzyl-5-phenylbarbituric acid, 1,3,5-trimethylbarbituric acid, sodium 1,3,5-trimethylbarbituric acid, calcium 1,3,5-trimethylbarbituric acid; tin compounds such as dibutyltin diacetate, dibutyltin dilaurate, dioctyltin dilaurate, dioctyltin diversate, dioctyltin bis(mercaptoacetic acid isooctyl ester) salt, and tetramethyl-1,3-diacetoxydistannoxane; aldehyde compounds such as lauryl aldehyde and terephthalaldehyde; and sulfur-containing compounds such as dodecyl mercaptan, 2-mercaptobenzoxazole, 1-decanethiol and thiosalicylic acid, but are not limited to.

In order to enhance photopolymerization promotion performances, it is effective to add, in addition to the above photopolymerization promoter, oxycarboxylic acids such as citric acid, malic acid, tartaric acid, glycolic acid, gluconic acid, α-oxyisobutyric acid, 2-hydroxypropanoic acid, 3-hydroxypropanoic acid, 3-hydroxybutanoic acid, 4-hydroxybutanoic acid and dimethylolpropionic acid, but are not limited to.

Examples of chemical polymerization initiator include redox type polymerization initiator systems comprising an peroxide/an amine compound, an peroxide/an amine compound/a aromatic sulfinic acid or a salt thereof or aromatic sulfonyl compounds, an peroxide/an amine compound/a (thio)barbituric acid compound or a (thio)barbituric acid compound, a peroxide/an amine compound/a borate compound, a peroxide/an ascorbic acid compound, a peroxide/a thiourea/a vanadium compounds or a copper compounds, an organometal type initiator systems that initiate polymerization by reacting with oxygen or water. Further examples include aromatic sulfinates, borate compounds, and (thio)barbiturates which can initiate polymerization by reacting with an acidic compound, but are not limited thereto.

Examples of the peroxide include sodium peroxodisulfate, potassium peroxodisulfate, ammonium peroxodisulfate, sodium peroxodiphosphate, potassium peroxodiphosphate, ammonium peroxodiphosphate, benzoyl peroxide, p-chlorobenzoyl peroxide, 2,4-dichlorobenzoyl peroxide, diacetyl peroxide, lauroyl peroxide, di-t-butyl peroxide, dicumyl peroxide, cumene hydroperoxide, t-butyl hydroperoxide, 1,1,3,3-tetra methyl butyl hydroperoxide, t-amyl hydroperoxide, iso-propylbenzene hydroperoxide, 5-phenyl-4-pentenyl hydroperoxide, t-butylperoxyisopropyl carbonate, methyl ethyl ketone peroxide, 1,1-bis(t-butylperoxy)cyclohexane, 1,1-bis(t-hexylperoxy)cyclohexane and t-butylperoxybenzoate, but are not limited to.

The amine compound is preferably an aromatic secondary or aromatic tertiary amine, and specific examples thereof include N-methyl-p-toluidine, N-(2-hydroxyethyl)-p-toluidine, ethyl p-methylaminobenzoate, N-methylaniline, N-(2-hydroxyethyl)aniline, N,N-dimethyl-p-toluidine, N,N-diethyl-p-toluidine, N,N-bis(2-hydroxyethyl)-p-toluidine, ethyl p-dimethylaminobenzoate, N,N-dimethylaniline, N,N-bis (2-hydroxyethyl)aniline, but are not limited to.

Examples of an aromatic sulfinic acid or a salt thereof or the aromatic sulfonyl compound include benzenesulfinic acid, p-toluenesulfinic acid, o-toluenesulfinic acid, 2,4,6-trimethylbenzenesulfinic acid, 2,4,6-triisopropylbenzenesulfinic acid, and its sodium salt, potassium salt, lithium salt or ammonium salt, benzenesulfonyl chloride, benzenesulfonyl fluoride, benzenesulfonamide, benzenesulfonyl hydrazide, p-toluene sulfonyl chloride, p-toluenesulfonyl fluoride, p-toluenesulfonamide, p-toluenesulfonyl hydrazide and the like, but are not limited to.

Examples of a (thio)barbituric acid compound or the (thio)barbituric acid salt compound include barbituric acid, 1,3-dimethylbarbituric acid, 1,3-diphenylbarbituric acid, 1,5-dimethylbarbituric acid, 5-butyl barbituric acid, 5-ethyl barbituric acid, 5-isopropyl barbituric acid, 5-cyclohexyl barbituric acid, 5-lauryl barbituric acid, 1,3,5-trimethyl barbituric acid, 1,3-dimethyl-5-ethyl barbituric acid, 1,3-dimethyl-n-butyl barbituric acid, 1,3-dimethyl-5-isobutyl barbituric acid, 1,3-dimethyl-5-cyclohexyl barbituric acid, 1,3-dimethyl-5-phenyl barbituric acid, 1-cyclohexyl-5-ethyl barbituric acid, 1-phenyl-5-benzyl barbituric acid, 1-benzyl-5-phenyl barbituric acid, thiobarbituric acid, 1,3-dimethylthiobarbituric acid, 5-phenylthiobarbituric acid and its alkali metal salts (lithium, sodium, potassium salts and the like), alkaline earth metal salts (calcium, strontium, barium salts and the like), ammonium salts, tetramethylammonium salts, and tetraethylammonium, but are not limited there to.

Examples of the borate compound include trialkylphenylboron, trialkyl (p-chlorophenyl) boron, trialkyl (p-fluorophenyl) boron, trialkyl (p-butylphenyl) boron, trialkyl (p-butyloxyphenyl) boron, monoalkyltriphenylboron, monoalkyltris (p-chlorophenyl) boron, monoalkyltris (p-fluorophenyl) boron, monoalkyltris (p-butylphenyl) boron, monoalkyltris (p-butyloxyphenyl) boron, tetraphenylboron, tetrakis (p-chlorophenyl) boron, tetrakis (p-fluorophenyl) boron, tetrakis (p-butylphenyl) boron, tetrakis (p-butyloxyphenyl) boron (wherein the alkyl group is n-butyl group, n-octyl group, n-dodecyl group and the like), and sodium salt, potassium salt, lithium salt, magnesium salt, tetramethylammonium salt, tetraethylammonium salt, tetrabutylammonium salt, methylpyridinium salt, ethylpyridinium salt, methylquinolinium and ethylquinolinium salt thereof and the like but are not limited to.

Examples of ascorbic acid compounds include L(+)-ascorbic acid, isoascorbic acid, L(+)-sodium ascorbate, L(+)-potassium ascorbate, L(+)-calcium ascorbate, sodium isoascorbate and the like, but are not limited to.

Examples of thiourea compounds include 1,3-dimethylthiourea, tetramethylthiourea, 1,1-diethylthiourea, 1,1,3,3-tetraethylthiourea, 1-allyl-2-thiourea, 1,3-diallylthiourea, 1,3-dibutylthiourea, 1,3-diphenyl-2-thiourea, 1,3-dicyclohexylthiourea, ethylenethiourea, N-methylthiourea, N-phenylthiourea, N-benzoylthiourea, N-acetylthiourea and the like, but are not limited to.

Examples of vanadium compounds include vanadium acetylacetonate, vanadyl acetylacetonate, vanadyl stearate, vanadium naphthenate, vanadium benzoylacetonate and the like, but are not limited to.

Examples of the copper compound include copper chloride, copper acetate, copper naphthenate, copper salicylate, copper gluconate, copper oleate, copper benzoate, copper acetylacetonate, and copper naphthenate and the like, but are not limited to.

Example of the organometal type polymerizable initiators may include organic boron compounds such as triphenylborane, tributylborane, and a partial oxide of tributylborane and the like, but are not limited to.

These polymerization initiators can be used not only singly but also in combinations of two or more, regardless of the polymerization manner or the polymerization method. In addition, there is no problem even if these polymerization initiators are subjected to a secondary treatment such as encapsulation in a microcapsule, if necessary.

The dental resin-reinforced glass ionomer cement composition (ii) in the present invention can contain an (I) acidic group-containing polymerizable monomer, if desired, in order to improve the adhesive property to the tooth substance, base metal, alumina, zirconia and the like. The (I) acidic group-containing polymerizable monomer can be the same as the acidic group-containing polymerizable monomer that can be used to obtain the (E) polymer of acidic group-containing polymerizable monomer. The (I) acidic group-containing polymerizable monomer may be used not only singly but also in combinations of a plurality thereof without any problem. In addition, in the case that the dental resin-reinforced glass ionomer cement composition (ii) is powder-liquid type, the (I) acidic group-containing polymerizable monomer may be compounded in at least one of the powder material and/or the liquid material. In the present invention, the (I) acidic group-containing polymerizable monomer refers to acidic group-containing polymerizable monomer other than (C) polymerizable monomer having acid anhydride structure and (J) 10-(meth)acryloyloxydecyl dihydrogen phosphate.

Among them, the (I) acidic group-containing polymerizable monomer is preferably a carboxy group-containing polymerizable monomer, and may have two or more carboxy groups. By containing a carboxy group-containing polymerizable monomer, it becomes easy to obtain a dental resin-reinforced glass ionomer cement composition (ii) having an excellent balance of adhesive property to tooth substance and mechanical properties.

The main components of the dental resin-reinforced glass ionomer cement composition (ii) in the present invention is described above the (D) acid-reactive glass powder, the (b) water, the (E) polymer of acidic group-containing polymerizable monomer, the (F) tri or more functional (meth)acrylamide-based polymerizable monomer, the (G) hydroxyl group-containing (meth)acrylate-based polymerizable monomer, and (H) polymerization initiator(I) acidic group-containing polymerizable monomer, and preferable contents are follows.

The content of the (D) acid-reactive glass powder may be 20 % by mass or more and 85 % by mass or less in 100 % by mass of the total mass of the dental resin-reinforced glass ionomer cement composition (ii) excluding the (H) polymerization initiator. When the content of the (D) acid-reactive glass powder is less than 20 % by mass, there is a case the mechanical strength of the hardened product may tend to decrease too much to cause problems in durability. When the content of acid-reactive glass powder exceeds 85 % by mass, there is a case that viscosity of the mixture may become higher to cause problem in operability. Further, there is a case that because the hardening rate became faster, a sufficient working time is not obtained.

The content of the (B) water in the case that the dental resin-reinforced glass ionomer cement composition (ii) is liquid-powder type may be 1 % by mass or more and 55 % by mass or less in 100 % by mass of the total mass of the liquid material excluding the (H) polymerization initiator. When the content of the (B) water is less than 1 % by mass, there is a case that the acid-base reaction is less likely to occur to cause hardening failure. When the content of the (B) water exceeds 55 % by mass, there is a case the mechanical strength of the hardened product may be too low to cause a problem in durability.

The content of the (B) water in the case that the dental resin-reinforced glass ionomer cement composition (ii) is two-paste type is preferably 1 % by mass or more and 30 % by mass or less, may be 5 % by mass or more and 25 % by mass or less in 100 % by mass of the total mass of the dental resin-reinforced glass ionomer cement composition (ii). When the content of the water is less than 1 % by mass, there is a case that the acid-base reaction is less likely to occur to cause hardening failure. When the content of the water exceeds 30 % by mass , there is a case the mechanical strength of the hardened product may be too low to cause a problem in durability.

The content of the (E) polymer of acidic group-containing polymerizable monomer may be 0.1 % by mass or more and 40 % by mass or less in 100 % by mass of the total mass of the dental resin-reinforced glass ionomer cement composition (ii) excluding the (H) polymerization initiator. When the content of the (E) polymer of acidic group-containing polymerizable monomer is less than 0.1 % by mass, there is a case that the acid-base reaction is less likely to occur to cause hardenning failure. When the content of the (E) polymer of acidic group-containing polymerizable monomer exceeds 40 % by mass, there is a case that viscosity of the mixture may become higher to cause problem in operability. Further, there is a case that because the hardenning rate became faster, a sufficient working time is not obtained.

The content of the (F) tri or more functional (meth)acrylamide-based polymerizable monomer may be 1 % by mass or more and 30 % by mass or less in 100 % by mass of the total mass of all polymerizable monomers, the (B) water and the (E) polymer of acidic group-containing polymerizable monomer in the dental resin-reinforced glass ionomer cement composition (ii). When the content of the (F) tri or more functional (meth)acrylamide-based polymerizable monomer is less than 1 % by mass, there is a case that the hardenability of the polymerizable monomer mixture may deteriorate and the mechanical properties may deteriorate. Further, there is a case where preservation stability may deteriorate. When the content of the (F) tri or more functional (meth)acrylamide-based polymerizable monomer exceeds 30 % by mass, there is a case that the compatibility of each polymerizable monomer, (B) water and the (E) polymer of acidic group-containing polymerizable monomer becomes poor and the hardened product becomes non-uniform, and therefore the mechanical properties and transparency may deteriorate.

The content of the (G) hydroxyl group-containing (meth)acrylate-based polymerizable monomer may be 3 % by mass or more and 60 % by mass or less in 100 % by mass of the total mass of all polymerizable monomers, the (B) water and the (E) polymer of acidic group-containing polymerizable monomer in the dental resin-reinforced glass ionomer cement composition (ii). When the content of the (G) hydroxyl group-containing (meth)acrylate-based polymerizable monomer is less than 3 % by mass, there is a case that the compatibility of each polymerizable monomer, (B) water and the (E) polymer of acidic group-containing polymerizable monomer becomes poor and the hardened product becomes non-uniform, and therefore the mechanical properties and transparency may deteriorate. When the content of the (G) hydroxyl group-containing (meth)acrylate-based polymerizable monomer exceeds 60 % by mass, there is a case that the hardenability of the polymerizable monomer mixture may deteriorate and the mechanical properties may deteriorate.

The (H) polymerization initiator may be added in the ratio of 0.01 % by mass or more and 10 % by mass or less with respect to 100 % by mass of the total mass of all polymerizable monomer in the dental resin-reinforced glass ionomer cement composition (ii). When the content of the (H) polymerization initiator is less than 0.01 % by mass, there is a case that the hardenability may deteriorate and the mechanical properties may deteriorate. When the content of the (H) polymerization initiator exceeds 10 % by mass, there is a case that preservation stability may deteriorate.

The content of the (I) acidic group-containing polymerizable monomer may be 1 % by mass or more and 20 % by mass or less in 100 % by mass of the total mass of all polymerizable monomer, the (b) water and the (E) polymer of acidic group-containing polymerizable monomer. When the content of the (I) acidic group-containing polymerizable monomer is less than 1 % by mass, there is a case that adhesive property to tooth substances deteriorates. When the content of the (I) acidic group-containing polymerizable monomer exceeds 20 % by mass, there is a case that the hardenability may deteriorate and the mechanical properties may deteriorate. Further, there is a case that preservation stability may deteriorate.

Furthermore, the dental resin-reinforced glass ionomer cement composition (ii) in the case of two-paste type in the present invention may contain a thickener to such a range that various properties are not adversely affected, for the purpose of adjusting pasty property. As the thickener which can be used in the dental resin-reinforced glass ionomer cement composition (ii) in the case of two-paste type in the present invention, any of an inorganic thickener and an organic thickener can be used.

Specific examples of an inorganic thickener include fumed silica, calcium carbonate, calcium silicate, magnesium silicate, and a clay mineral such as saponite, montmorillonite, beidellite, vermiculite, sauconite, stevensite, hectorite, smectite, nekutaito and sepiolite, but are not limited to.

Specific examples of an organic thickener include methyl cellulose, hydroxyethyl cellulose, hydroxymethyl cellulose, hydroxypropyl cellulose, carboxymethyl cellulose, sodium carboxymethyl cellulose, calcium carboxymethyl cellulose, carboxypolymethylene, sodium alginate, propylene glycol alginate ester, sodium polyacrylate, starch, starch sodium glycolate, starch phosphate ester, polyvinyl pyrrolidone, carboxyvinyl polymer, khaya gum, arabic gum, karaya gum, guar gum, but are not limited to. These thickeners may be used alone or as a mixture of two or more thereof.

The content of the thickener in each paste may be within a range of 0.1 to 20.0 % by mass.

The dental resin-reinforced glass ionomer cement composition (ii) in the present invention may contain other polymerizable monomer other than the (F) tri or more functional (meth)acrylamide-based polymerizable monomer, the(G) hydroxyl group-containing (meth)acrylate-based polymerizable monomer and the (I) acidic group-containing polymerizable monomer to such a range that various properties are not adversely affected, for the purpose of improving mechanical properties. As such polymerizable monomer, known polymerizable monomers may be used regardless of the number (monofunctional or multifunctional) or the type of radical polymerizable unsaturated groups. Specific examples of the polymerizable monomer having a (meth) acryloyl group as an unsaturated group are listed below as representative example.

Examples of the monofunctional polymerizable monomer include methyl(meth)acrylate, ethyl(meth)acrylate, butyl(meth)acrylate, hexyl(meth)acrylate, glycidyl(meth)acrylate, lauryl(meth)acrylate, cyclohexyl(meth)acrylate, allyl(meth)acrylate, 2-ethoxyethyl(meth)acrylate, methoxypolyethyleneglycol(meth)acrylate, isopropyl(meth)acrylate, isobutyl(meth)acrylate, 2-ethylhexyl(meth)acrylate, tetrahydrofurfuryl(meth)acrylate, benzyl(meth)acrylate, isobornyl(meth)acrylate and the like, but are not limited to.

Examples of the aromatic bifunctional polymerizable monomer include 2,2-bis(4-(meth)acryloyloxyphenyl)propane, 2,2-bis(4-(meth)acryloyloxyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2(4-(meth)acryloyloxyethoxyphenyl)-2(4-(meth)acryloyloxydiethoxyphenyl)propane, 2(4-(meth)acryloyloxydiethoxyphenyl)-2(4-(meth)acryloyloxytriethoxyphenyl)propane, 2(4-(meth)acryloyloxydipropoxyphenyl)-2(4-(meth)acryloyloxytriethoxyphenyl)propane, 2,2-bis(4-(meth)acryloyloxydipropoxyphenyl)propane, 2,2-bis(4-(meth)acryloyloxyisopropoxyphenyl)propane, 2,2-bis(4-(meth)acryloyloxypolyethoxyphenyl)propane, 9,9-bis[4-(2-acryloyloxyethoxy)phenyl]fluorine and the like, but are not limited to.

Examples of the aliphatic bifunctional polymerizable monomer include ethyleneglycoldi(meth)acrylate, diethyleneglycoldi(meth)acrylate, triethyleneglycoldi(meth)acrylate, tetraethyleneglycoldi(meth)acrylate, polyethyleneglycoldi(meth)acrylate, neopentylglycoldi(meth)acrylate, propyleneglycoldi(meth)acrylate, dipropylene glycol di(meth)acrylate, tripropylene glycol di(meth)acrylate, tricyclodecane dimethanol di(meth)acrylate, 1,3-butanediol di(meth)acrylate, 1,4-butanediol di(meth)acrylate, 1,6-hexanediol di(meth)acrylate, 1,9-nonanediol di(meth)acrylate, 1,10-decanediol di(meth)acrylate and the like, but are not limited to.

Examples of the tri functional polymerizable monomer include trimethylolpropane tri(meth)acrylate, trimethylolethane tri(meth)acrylate, and trimethylolmethan tri(meth)acrylate, but are not limited to.

Examples of a tetra functional polymerizable monomer include pentaerythritol tetra(meth)acrylate and ditrimethylolporpane tetra (meth)acrylate, but are not limited to.

Examples of a urethane type polymerizable monomer include di(meth)acrylates having a bifunctional or tri or higher functional urethane bond, derived from an adduct of a hydroxyl group-containing polymerizable monomer such as 2-hydroxyethyl (meth)acrylate, 2-hydroxypropyl (meth)acrylate and 3-chloro-2-hydroxypropyl (meth)acrylate and a diisocyanate compound such as methylcyclohexane diisocyanate, methylenebis (4-cyclohexylisocyanate), hexamethylene diisocyanate, trimethylhexamethylene diisocyanate, isophorone diisocyanate, diisocyanate methylbenzene and 4,4-diphenylmethane diisocyanate, but are not limited to.

In addition to the above described (meth)acrylate group-containing polymerizable monomer, a polymerizable monomer having a sulfur atom in the molecule, a polymerizable monomer having a fluoro group, and an oligomer or polymer having at least one polymerizable group can be used. These polymerizable monomer may be used alone or in combination of two or more as necessary.

Further, a polymerizable monomer having one or two (meth)acrylamide groups in the molecule can be contained in the dental resin-reinforced glass ionomer cement composition (ii) in the present invention to such a range that various properties are not adversely effected, without any problem.

In the case that the dental resin-reinforced glass ionomer cement composition (ii) is powder-liquid type, the content of the other polymerizable monomer other than the (F) tri or more functional (meth)acrylamide-based polymerizable monomer, the (G) hydroxyl group-containing (meth)acrylate-based polymerizable monomer and the (I) acidic group-containing polymerizable monomer may be within a range of 5.0 % by mass or less in 100 mass % of the total mass of the dental resin-reinforced glass ionomer cement composition (ii).

In the case that the dental resin-reinforced glass ionomer cement composition (ii) is two-paste type, the content of the other polymerizable monomer other than the (F) tri or more functional (meth)acrylamide-based polymerizable monomer, the (G) hydroxyl group-containing (meth)acrylate-based polymerizable monomer and the (I) acidic group-containing polymerizable monomer may be within a range of 10 % by mass or less in 100 mass % of the total mass of the dental resin-reinforced glass ionomer cement composition (ii).

For the purpose of controlling the acid-base reaction of the (D) acid-reactive glass powder and the (E) polymer of acidic group-containing polymerizable monomer, and adjusting the working time and hardening time, the dental resin-reinforced glass ionomer cement composition (ii) in the present invention may contain a polybasic carboxylic acid, a phosphoric acid, a pyrophosphoric acid or a tripolyphosphoric acid, but are not limited to.

Specific examples of the polybasic carboxylic acid used in the dental resin-reinforced glass ionomer cement composition (ii) in the present invention include tartaric acid, citric acid, maleic acid, fumaric acid, malic acid, aconitic acid, tricarballylic acid, itaconic acid, 1-butene-1,2,4-tricarboxylic acid, and 3-butene-1,2,3-tricarboxylic acid, and the like. The aforementioned polybasic carboxylic acid are not limited to these, but can be used without any limitation.

A polybasic carboxylic acid, a phosphoric acid, a pyrophosphoric acid and/or a tripolyphosphoric acid may be used alone or in combination of two or more thereof. A content of the polybasic carboxylic acid, the phosphoric acid, the pyrophosphoric acid and/or the tripolyphosphoric acid is preferably in the range from 0.1 mass % to 15.0 mass % in 100 mass % of the total mass of the dental resin-reinforced glass ionomer cement composition (ii).

Further, a surfactant can be contained in the dental resin-reinforced glass ionomer cement composition (ii) in the present invention to such a range that various properties are not adversely affected, for the purpose of improving mixability. The surfactant which can be used in the dental resin-reinforced glass ionomer cement composition (ii) in the present invention may be any of an ionic surfactant and a nonionic surfactant.

Specific examples of the anionic surfactant in the ionic surfactant include aliphatic carboxylic acid metal salts such as sodium stearate, sulfated aliphatic carboxylic acid metal salts such as sodium dioctyl sulfosuccinate, and metal salts of higher alcohol sulfate ester such as sodium stearyl sulfate.

In addition, examples of the cationic surfactant include an adduct of higher alkylamine and ethylene oxide, amines made from lower amine, and alkyltrimethylammonium salts such as lauryltrimethylammoniun chloride. Further, examples of the amphoteric surfactant include metal salts of higher alkylaminopropionic acid such as sodium stearylaminopropionate, and betaines such as lauryldimethylbetaine.

Examples of the nonionic surfactant include polyethylene glycol type and polypropylene glycol type in which ethylene oxide or propylene oxide is added to higher alcohols, alkyl phenols, fatty acids, higher fatty amines, or aliphatic amides, and polyhydric alcohol type in which a fatty acid are ester bonded to polyhydric alcohols, diethanolamines, or saccharides.

The aforementioned surfactants are not limited to these, but can be used without any limitation. These surfactants can be used alone or in a combination of a few kinds. The content of the surfactant may be 0.001 mass % to 5.0 mass % in 100 mass % of the total mass of the dental resin-reinforced glass ionomer cement composition (ii).

Further, a non-acid reactive powder can be contained in the dental resin-reinforced glass ionomer cement composition (ii) in the present invention to such a range that various properties are not adversely affected, for the purpose of adjusting operability, a mechanical characteristic or a hardening characteristic.

As the non-acid reactive powder used in the dental resin-reinforced glass ionomer cement composition (ii) in the present invention, any non-acid reactive powder as long as the non-acid reactive powder not containing element which may react with an acid group of the (E) polymer of acidic group-containing polymerizable monomer can be used without any limitation.

Examples of the non-acid reactive powder include known dental fillers such as an inorganic filler, an organic filler and an organic-inorganic composite filler, and these can be used alone or in a combination of a few of them without any limitation. Among them, an inorganic filler may be used. In addition, a shape of these non-acid reactive powder is not particularly limited, but arbitral particle shapes such as spherical, needle-like, plate-like, ground-like, and scaly-shapes and aggregate thereof may be used, but is not limited thereto. An average particle diameter of the non-acid reactive powder is not particular limited, but may be within a range of 0.001 to 30 µm.

Specific examples of the inorganic filler include quartz, amorphous silica, ultrafine silica, various glasses which does not contain element which may react with an acid group (including a glass by melting method, synthetic glass by sol-gel method, a glass produced by a vapor phase reaction, and the like), silicon nitride, silicon carbide, boron carbide and the like, but are not limited thereto.

The content of the non-acid reactive powder may be 0.001 mass % to 40 mass % in 100 mass % of the total mass of the dental resin-reinforced glass ionomer cement composition (ii).

In the dental tooth surface treatment material composition (i) and the dental resin-reinforced glass ionomer cement composition(ii) in the present invention, heretofore known various additives can be arbitrarily mixed if necessary. Examples of such additives which can be used in the present invention include a polymerization inhibitor, a chain transfer agent, a colorant, a discoloration preventing agent, a fluorescent agent, an ultraviolet absorber, an antibacterial agent and an antiseptic agent.

The method of using the dental resin-reinforced glass ionomer cement kit of the present invention is as shown below, but the present invention is not limited to these procedures.

That is, the method contains, a first step in which the dental tooth surface treatment material composition (i) of the present invention is applied to the tooth surface after removing caries, foriming cavity and the like and washing and drying, a second step in which the applied surface is dried immediately without washing with water, a third step in which the dried surface is applied with the dental resin-reinforced glass ionomer cement composition (ii) without irradiating light, and a fourth step in which the applied dental resin-reinforced glass ionomer cement composition (ii) is cured by irradiating light. As described above, the dental resin-reinforced glass ionomer cement kit of the present invention does not require water washing and polymerization by light irradiation for the dental tooth surface treatment material composition (i) applied to the tooth surface.

The dental resin-reinforced glass ionomer cement kit of the present invention can be used in a wide range of applications in dental treatment, such as pit and fissure sealant, lining material and abutment construction material, in addition to being used as filling material and luting materials.

### [Examples]

Hereinafter, the present invention will be described in detail with reference to Examples and Comparative Examples. However, the present invention is not limited to these Examples. The components (A) to (J) and other components used for preparing the dental tooth surface treatment material composition (i) and the dental resin-reinforced glass ionomer cement composition (ii) of Examples and Comparative Examples, and their abbreviations are as follows.
[(A) Water-soluble organic solvent]
   Ethanol
   Acetone
   ·Propylene glycol
[(B) Water]
   ·Distilled water
[(C) Polymerizable monomer having acid anhydride structure]
   ·4-META: 4-methacryloyloxyethyl trimellitic anhydride
   ·4-AETA: 4-acryloyloxyethyl trimellitic anhydride
[(D) Acid-reactive glass powder]
   ·CK-Si-1: Silane-treated fluoroaluminosilicate glass powder 1
   (50% particle diameter: 4.5 um)
[(E) Polymer of acidic group-containing polymerizable monomer]
   ·PCA1: acrylic acid homopolymer powder (weight average molecular weight: 50,000)
[(F) Trifunctional or higher (meth)acrylamide-based polymerizable monomer]
   Tetrafunctional acrylamide polymerizable monomer
      ·FAM-401 (manufactured by Fujifilm Corporation): a compound represented by the formula (4) in which all R¹ are hydrogen atoms.
   Trifunctional acrylamide polymerizable monomer
      ·FAM-302L (manufactured by Fujifilm Corporation): a compound represented by the formula (8)
[(G) Hydroxyl group-containing (meth)acrylate-based polymerizable monomer]
   ·HEMA: 2-hydroxyethyl methacrylate
   ·Bis-GMA: bisphenol A diglycidyl methacrylate
   ·GDMA: glycerine dimethacrylate
[(H) Polymerization initiator]
   ·CQ: dl-camphorquinone
   ·DMBE: Ethyl p-dimethylaminobenzoate
   ·DM-3B: Dimethylaminoethyl methacrylate
   ·p-TSNa: Sodium p-toluenesulfinate
   ·KPS: Potassium peroxodisulfate
   ·AA: Ascorbic acid
   ·DEPT: N,N-bis(2-hydroxyethyl)-p-toluidine
[(I) Acidic group-containing polymerizable monomer]
   ·4-MET: 4-methacryloyloxyethyl trimellitic acid
   ·4-AET: 4-acryloyloxyethyl trimellitic acid
[(J) 10-(meth)acryloyloxydecyl dihydrogen phosphate]
   ·10-MDP: 10-methacryloyloxydecyl dihydrogen phosphate

[Others]
·14EG: Polyethylene glycol #600 dimethacrylate
·HEAA: Hydroxyethyl acrylamide
·2AM: N,N'-methylenebismethacrylamide
·PEG400: Polyethylene glycol
Aerosil R972: Fumed silica average particle diameter about 16 nm

The preparing method of the silane-treated fluoroaluminosilicate glass powder is as follows.

### [Preparation of silane-treated fluoroaluminosilicate glass powder 1]

Various raw materials: silicon dioxide, aluminum oxide, aluminum phosphate, sodium fluoride, and strontium carbonate (glass composition: SiO₂: 26.4 % by mass, Al₂O₃: 29.3 % by mass, SrO: 20.5 % by mass, P₂O₅: 10.9 % by mass, Na₂O: 2.5 % by mass, and F: 10.4 % by mass) were mixed and the mixed material was molten at 1400 °C in a melting furnace. The melt was taken out from the melting furnace and was quenched in water to prepare a glass. The resulting glass was pulverized to obtain fluoroaluminosilicate glass powder 1. The glass powder was measured for 50 % particle diameter by a laser diffraction type grain size measuring apparatus (Microtrac MT3300EXII: NIKKISO Co., Ltd.). The result was 4.5 pm. The fluoroaluminosilicate glass powder 1 of 200 g was dispersed in 500 mL of water, 2 g of 3-methacryloyloxypropyltrimethoxysilane was added, and the mixture was stirred at room temperature for 2 hours. After the solvent was distilled off under reduced pressure, it was further dried at 100 °C for 5 hours to obtain silane-treated fluoroaluminosilicate glass powders 1.

### [Preparation of dental tooth surface treatment material composition]

The dental tooth surface treatment material compositions of Examples and Comparative Examples were prepared by mixing each component in the ratios shown in Tables 1 to 2.

### [Preparation of powder material, liquid material and paste material for dental resin-reinforced glass ionomer cement composition]

The components were mixed in the ratios shown in Tables 3 to 6 to prepare powder materials/liquid materials and paste materials of the dental resin-reinforced glass ionomer cement composition used in Examples and Comparative Examples. The combinations of the dental resin-reinforced glass ionomer cement composition are shown in Table 7.

**[Table 1]**

| | | | | | | | | | | | | | (% by mass) | |
|---|---|---|---|---|---|---|---|---|---|---|---|---|---|---|
| Dental tooth surface treatment material composition (i) | | C-1 | C-2 | C-3 | C-4 | C-5 | C-6 | C-7 | C-8 | C-9 | C-10 | C-11 | C-12 | C-13 |
| (A) water-soluble organic solvent | Ethanol | 20 | 70 | | | 53 | | | | 69 | | | | |
| | Acetone | | | 20 | 70 | | 38 | 60 | 20 | | 40 | 79 | | 45 |
| | Propylene glycol | | | | | | | | | | | | 70 | |
| (B) water | Distilled water | 70 | 20 | 70 | 20 | 43.8 | 38 | 20 | 79 | 20 | 20 | 20 | 20 | 41.9 |
| (C) polymerizable monomer having acid anhydride structure | 4-META | | | 10 | 10 | 3 | 20 | | | 1 | 30 | 1 | 10 | 10 |
| | 4-AETA | 10 | 9.9 | | | | | 20 | 1 | | | | | |
| (I) acidic group-containing polymerizable monomer | 4-MET | | | | | | | | | | | | | 2 |
| (J) 10-(meth)acryloyloxydecyl dihydrogen phosphate | 10-MDP | | | | | 0.1 | 3.9 | | | | 9.9 | | | 1 |
| (H) polymerization initiator | CQ | | 0.1 | | | 0.1 | 0.1 | | | 1 | 0.1 | | | 0.1 |
| | DM-3B | | | | | | | | | 9 | | | | |

**[Table 2]**

| | | | | | | | | | | (% by mass) | |
|---|---|---|---|---|---|---|---|---|---|---|---|
| Dental tooth surface treatment material composition (i) | | C-14 | C-15 | C-16 | C-17 | C-18 | C-19 | C-20 | C-21 | C-22 | C-23 |
| (A) water-soluble organic solvent | Ethanol | 15 | 65 | | 50 | 60 | | 84 | 15 | 79 | 40 |
| (B) water | Distilled water | 79 | 15 | 40 | 49 | 30 | 40 | 15 | 84 | 10 | 20 |
| (C) polymerizable monomer having acid anhydride structure | 4-META | 1 | | | 0.5 | | 10 | 1 | 1 | 7 | 40 |
| (I) acidic group-containing polymerizable monomer | 4-MET | | 20 | 19 | | | | | | | |
| (J) 10-(meth)acryloyloxydecyl dihydrogen phosphate | 10-MDP | 4 | | 1 | | 9.9 | | | | 3 | |
| (H) polymerization initiator | CQ | 1 | | | 0.5 | 0.1 | | | | 1 | |
| Others | PEG400 | | | 40 | | | 50 | | | | |

**[Table 3]**

| | | | (% by mass) | |
|---|---|---|---|---|
| Powder material | | Powder material P1 | Powder material P2 | Powder material P3 |
| (D) acid-reactive glass powder | CK-Si-1 | 99.75 | 98.98 | 95 |
| (E) polymer of acidic group-containing polymerizable monomer | PCA1 | | | 1.65 |
| (H) polymerization initiator | CQ | | | 0.1 |
| | p-TSNa | | 1 | 0.2 |
| | KPS | 0.05 | 0.01 | |
| | DMBE | 0.2 | | |
| | AA | | 0.01 | 0.05 |
| (I) acidic group-containing polymerizable monomer | 4-MET | | | 3 |

**[Table 4]**

| | | | | | | | (% by mass) | |
|---|---|---|---|---|---|---|---|---|
| Liquid material | | Liquid material L1 | Liquid material L2 | Liquid material L3 | Liquid material L4 | Liquid material L5 | Liquid material L6 | Liquid material L7 |
| (B) water | Distilled water | 1 | 45 | 30 | 23.5 | 24.7 | 55 | 16.7 |
| (E) polymer of acidic group-containing polvmerizable monomer | PCA1 | 1 | 45 | 20 | 11.8 | 15 | 21.7 | 7 |
| (F) tri or more functional (meth)acrylamide-based polymerizable monomer | FAM-401 | | 1 | 10 | | 10 | | |
| | FAM-302L | 30 | | | 17.6 | | 5 | 10 |
| (G) hydroxyl group-containing (meth)acrylate-based polymerizable monomer | HEMA | 30 | 8 | 31.7 | 23.5 | 20 | 12 | 37 |
| | Bis-GMA | 3 | | | | | | 1 |
| | GDMA | 14.9 | | 8 | 11.8 | 20 | 3 | 25 |
| (H) polymerization initiator | CQ | 0.1 | 0.2 | 0.3 | 0.3 | 0.3 | 0.3 | 0.3 |
| | DMBE | | | | 1 | | | |
| | DM-3B | | 0.8 | | | | | |
| (I) acidic group-containing polymerizable monomer | 4-MET | 20 | | | 10.5 | | 3 | 3 |
| | 4-AET | | | | | 10 | | |

**[Table 5]**

| | | | | (% by mass) | |
|---|---|---|---|---|---|
| Liquid material | | Liquid material L8 | Liquid material L9 | Liquid material L10 | Liquid material L11 |
| (B) water | Distilled water | 30 | 30 | 30 | 30 |
| (E) polymer of acidic group-containing polymerizable monomer | PCA1 | 19.7 | 19.7 | 19.7 | 19.7 |
| (F) tri or more functional (meth)acrylamide-based polymerizable monomer | FAM-401 | | | | |
| | FAM-302L | | | | |
| (G) hydroxyl group-containing (meth)acrylate-based polymerizable monomer | HEMA | 20 | | 20 | |
| | Bis-GMA | | | | |
| | GDMA | 10 | | 10 | |
| (H) polymerization initiator | CQ | 0.3 | 0.3 | 0.3 | 0.3 |
| | DMBE | | | | |
| | DM-3B | | | | |
| (I) acidic group-containing polymerizable monomer | 4-MET | 10 | 10 | | |
| | 4-AET | | | | |
| Others | 14EG | | 40 | | |
| | HEAA | | | 20 | 30 |
| | 2AM | 10 | | | 20 |

**[Table 6]**

| | | | | (% by mass) | |
|---|---|---|---|---|---|
| Paste used in Examples | | Paste 1 | Paste 2 | Paste 3 | Paste 4 |
| (B) water | Distilled water | | 23 | | 50 |
| (D) acid-reactive glass powder | CK-Si-1 | 60 | | 15 | |
| (E) polymer of acidic group-containing polymerizable monomer | PCA1 | | 20 | | 20 |
| (F) tri or more functional (meth)acrylamide-based polymerizable monomer | FAM-401 | | 5 | 5 | |
| | FAM-302L | | 4.6 | | 5 |
| (G) hydroxyl group-containing (meth)acrylate-based polymerizable monomer | HEMA | 17.5 | 30 | 40 | 10 |
| | GDMA | 17.2 | 5 | | 5 |
| | Bis-GMA | | 2 | 30 | |
| (H) polymerization initiator | CQ | 0.3 | 0.3 | | 0.3 |
| | DEPT | 0.5 | | 0.5 | |
| | DMBE | | | 0.5 | |
| | AA | | | | |
| (I) acidic group-containing polymerizable monomer | 4-MET | | 10 | | |
| | 4-AET | | | | 9 |
| Others | Aerosil R972 | 4.5 | 0.1 | 9 | 0.7 |

**[Table 7]**

| | | | | | | | | | | | | | |
|---|---|---|---|---|---|---|---|---|---|---|---|---|---|
| Dental resin-reinforced glass ionomer cement comnosition (ii) | R-1 | R-2 | R-3 | R-4 | R-5 | R-6 | R-7 | R-8 | R-9 | R-10 | R-11 | R-12 | R-13 |
| First material | Powder material P1 | Powder material P2 | Powder material P3 | Powder material P2 | Powder material P2 | Powder material P2 | Powder material P3 | Powder material P2 | Powder material P2 | Paste 1 | Paste 3 | Powder material P2 | Powder material P2 |
| Second material | Liquid material P2 | Liquid material P1 | Liquid material P3 | Liquid material P4 | Liquid material P5 | Liquid material P8 | Liquid material P9 | Liquid material P10 | Liquid material P11 | Paste 2 | Paste 4 | Liquid material P6 | Liquid material P7 |
| Ratio of First material and Second material (First material: Second material) | 3.0 : 1.0 | 3.0 : 1.0 | 3.0 : 1.0 | 3.0 : 1.0 | 3.0 : 1.0 | 3.0 : 1.0 | 3.0 : 1.0 | 3.0 : 1.0 | 3.0 : 1.0 | 1.0 : 1.0 | 0.6 : 1.2 | 3.0 : 1.0 | 3.0 : 1.0 |

The dental resin-reinforced glass ionomer cement kits in which the dental tooth surface treatment material composition (C1 to C23) and the dental resin-reinforced glass ionomer cement composition (R1 to R13 ) are combined as shown in Tables 8 and 9 were evaluated for applicability, drying property, shear adhesive strength to enamel and dentin, and storage stability. In addition, a commercially available dental filling composite resin (LITE-FIL II: manufactured by SHOFU INC.) was also evaluated for shear adhesion strength to enamel and dentin (Comparative Example 11). These test results are shown in Tables 8 and 9. The evaluation method is as shown below.

### <Applicability>

The labial surfaces of bovine mandibular incisor was polished with #80 silicon carbide paper (manufactured by Nihon Kenshi Co., Ltd.) under running water to expose a flat dentin surface, and thereafter the flat dentin surface was polished with #600 silicon carbide paper (manufactured by Nihon Kenshi Co., Ltd.) under running water to obtain a smooth surface. The applicability in applying the dental tooth surface treatment material composition to the smooth surface after washing with water and drying was evaluated. The evaluation criteria are as follows.
A: Easy to apply and a uniform applied surface was obtained.
B: It is sticky and difficult to apply and the applied surface was uneven.

Evaluation "A" means good applicability.

### <Drying property>

The labial surfaces of bovine mandibular incisor was polished with #80 silicon carbide paper (manufactured by Nihon Kenshi Co., Ltd.) under running water to expose a flat dentin surface, and thereafter the flat dentin surface was polished with #600 silicon carbide paper (manufactured by Nihon Kenshi Co., Ltd.) under running water to obtain a smooth surface. The drying property in air-drying at a discharge pressure of 0.05 MPa for 5 seconds immediately after application of the dental tooth surface treatment material composition to the smooth surface after washing with water and drying was evaluated. The evaluation criteria are as follows.
A: Almost no stickiness remains on the dry surface.
B: Stickiness remains on the dry surface.

Evaluation "A" means good drying property.

<Shear bond strength to enamel and dentin>

The labial surfaces of bovine mandibular incisor was polished with #80 silicon carbide paper (manufactured by Nihon Kenshi Co., Ltd.) under running water to expose a flat surface of enamel or dentin, and thereafter the flat surface were polished with #600 silicon carbide paper (manufactured by Nihon Kenshi Co., Ltd.) under running water to obtain smooth surfaces. A silicon ring having a thickness of 2 mm and having a cavity of Φ4 mm was placed on the smooth surface after washing with water and drying to define an adhesion area.

Immediately after applying the dental tooth surface treatment composition to the tooth surface in the cavity, air drying was performed at a discharge pressure of 0.05 MPa for 5 seconds. Then, after filling with the dental resin-reinforced glass ionomer cement composition, an adhesion test specimen were prepared by hardening by light irradiation for 10 seconds using a photopolymerization irradiator (PEN Bright, manufactured by SHOFU INC.). The number of the prepared adhesion test specimen was five.

After the adhesion test specimen was allowed to stand in a constant temperature water bath at 37 °C and a humidity of 90 % or higher for 60 minutes, it was immersed in distilled water at 37 °C for 24 hours. After removing the silicon ring, the shear bond strength of the adhesion test specimen was measured using a universal tester (manufactured by Shimadzu Corporation) at a crosshead speed of 1 mm/min. The values in the table represent the average values of five adhesive test specimens. When the adhesion strength to both the enamel and the dentin was 5 MPa or more, it was evaluated that the adhesion strength is good.

### <Storage stability>

After storing the dental tooth surface treatment material composition in a thermostat at 50 °C for 2 months, the applicability was evaluated according to the test method of <Applicability> above, and the result was compared with the applicability of the dental tooth surface treatment material composition immediately after preparation. The evaluation criteria are as follows.
A: Almost no change in applicability.
B: Adhesiveness was increased, making it difficult to apply.
C: Separation or gelation of the liquid material had occurred and it cannot be used.

Evaluation "A" means good Storage stability.

**[Table 8]**

| | Example 1 | Example 2 | Example 3 | Example 4 | Example 5 | Example 6 | Example 7 |
|---|---|---|---|---|---|---|---|
| Dental tooth surface treatment material composition (i) | C-1 | C-2 | C-3 | C-4 | C-5 | C-6 | C-7 |
| Dental resin-reinforced glass ionomer cement composition (ii) | R-1 | R-2 | R-3 | R-4 | R-11 | R-5 | R-5 |
| Applicability of Dental tooth surface treatment material | A | A | A | A | A | A | A |
| Drying property of Dental tooth surface treatment material | A | A | A | A | A | A | A |
| Shear bond strength to enamel (Mpa) | 9.1 | 6.7 | 8.6 | 7.7 | 6.5 | 19.1 | 15.4 |
| Shear bond strength to dentin (Mpa) | 7.2 | 6.6 | 8.9 | 8.0 | 6.3 | 13.9 | 11.1 |
| Storage stability | A | A | A | A | A | A | A |

| | Example 8 | Example 9 | Example 10 | Example 11 | Example 12 | Example 13 | |
|---|---|---|---|---|---|---|---|
| Dental tooth surface treatment material composition (i) | C-8 | C-9 | C-10 | C-11 | C-12 | C-13 | |
| Dental resin-reinforced glass ionomer cement composition (ii) | R-5 | R-10 | R-5 | R-5 | R-12 | R-13 | |
| Applicability of Dental tooth surface treatment material | A | A | A | A | A | A | |
| Drying property of Dental tooth surface treatment material | A | A | A | A | A | A | |
| Shear bond strength to enamel (Mpa) | 5.7 | 5.6 | 18.3 | 5.5 | 6.2 | 12.1 | |
| Shear bond strength to dentin (Mpa) | 6.5 | 6.1 | 12.7 | 5.9 | 7.1 | 9.9 | |
| Storage stability | A | A | A | A | A | A | |

**[Table 9]**

| | Comparative Example 1 | Comparative Example 2 | Comparative Example 3 | Comparative Example 4 | Comparative Example 5 | Comparative Example 6 | Comparative Example 7 | Comparative Example 8 |
|---|---|---|---|---|---|---|---|---|
| Dental tooth surface treatment material composition (i) | C-14 | C-15 | C-16 | C-17 | C-18 | C-19 | C-10 | C-10 |
| Dental resin-reinforced glass ionomer cement composition (ii) | R-5 | R-5 | R-5 | R-5 | R-5 | R-5 | R-6 | R-7 |
| Applicability of Dental tooth surface treatment material | B | A | B | A | A | B | A | A |
| Drying property of Dental tooth surface treatment material | A | A | B | A | A | B | A | A |
| Shear bond strength to enamel (Mpa) | 4.7 | 6.5 | 4.0 | 3.9 | 4.5 | 4.8 | 6.8 | 5.3 |
| Shear bond strength to dentin (Mpa) | 4.1 | 4.5 | 3.8 | 4.0 | 4.2 | 4.9 | 4.7 | 4.8 |
| Storage stability | C | C | B | A | A | A | A | A |

| | Comparative Example 9 | Comparative Example 10 | Comparative Example 11 | | Comparative Example 12 | Comparative Example 13 | Comparative Example 14 | Comparative Examule 15 |
|---|---|---|---|---|---|---|---|---|
| Dental tooth surface treatment material composition (i) | C-3 | C-3 | C-10 | | C-20 | C-21 | C-22 | C-23 |
| Dental resin-reinforced glass ionomer cement composition (ii) | R-β | R-9 | LITE-FIL II | | R-5 | R-5 | R-5 | R-5 |
| Applicability of Dental tooth surface treatment material | A | A | - | | A | A | A | B |
| Drying property of Dental tooth surface treatment material | A | A | - | | A | A | A | B |
| Shear bond strength to enamel (Mpa) | 4.5 | 4.2 | 0.0 | | 4.0 | 4.2 | 5.1 | 9.8 |
| Shear bond strength to dentin (Mpa) | 4.1 | 3.9 | 0.0 | | 3.7 | 3.8 | 4.1 | 8.1 |
| Storage stability | A | A | - | | A | B | A | A |

As shown in Table 8, the dental resin-reinforced glass ionomer cement kits of Examples 1 to 13 were excellent in the applicability and drying property of the dental tooth surface treatment material (i), and exhibited high adhesive strength. In addition, even after storing at 50 °C for 2 months, the applicability immediately after preparation was maintained. On the other hand, as shown in Table 9, the dental resin-reinforced glass ionomer cement kits of Comparative Examples 1 to 15 and the commercially available dental composite resin (Comparative Example 11) were inferior to the dental resin-rein forced glass ionomer cement compositions of Examples 1 to 13 in any of properties such as applicability, drying property, adhesive strength, and storage stability.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

The dental resin-reinforced glass ionomer cement kit of the present invention can be used for filling and restoring a tooth in which a form was partially lost by caries breakages and the like and for luting a dental prosthesis device to a tooth in which a form was lost.

## Claims

1. A dental resin-reinforced glass ionomer cement kit comprising a dental tooth surface treatment material composition (i) and a dental resin-reinforced glass ionomer cement composition (ii), wherein
the dental tooth surface treatment material composition (i) contains
(A) water-soluble organic solvent: 20 % by mass or more and 79 % by mass or less,
(B) water: 20 % by mass or more and 79 % by mass or less, and
(C) polymerizable monomer having acid anhydride structure: 1 % by mass or more and 30 % by mass or less, and
the dental resin-reinforced glass ionomer cement composition (ii) contains
(D) acid-reactive glass powder,
(B) water,
(E) polymer of acidic group-containing polymerizable monomer,
(F) tri or more functional (meth)acrylamide-based polymerizable monomer,
(G) hydroxyl group-containing (meth)acrylate-based polymerizable monomer, and
(H) polymerization initiator.

2. The dental resin-reinforced glass ionomer cement kit according to claim 1, wherein
the (C) polymerizable monomer having acid anhydride structure is a compound represented by formula (1): 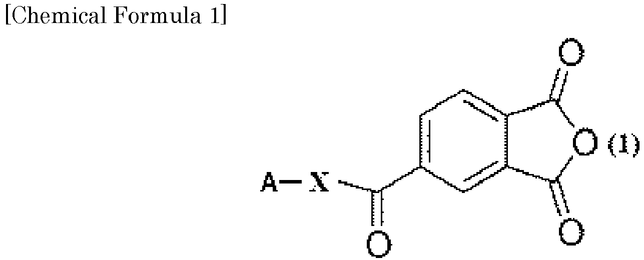 wherein X represents a linear or branched alkylene group having 1 to 10 carbon atoms which may have an ether bond, an amide bond, an ester bond and/or a substituent, and A is (meth)acryloyloxy group or (meth)acrylamide group.

3. The dental resin-reinforced glass ionomer cement kit according to claim 1 or 2, wherein
the (C) polymerizable monomer having acid anhydride structure is at least one selected from the group consisting of 4-(meth)acryloyloxyethyl trimellitic anhydride, 4-(meth)acryloylaminoethyl trimethic anhydride, 4-(meth)acryloyloxyethyl trimellitic acid amide anhydride, 4-(meth)acryloylaminoethyltrimethic acid amide anhydride.

4. The dental resin-reinforced glass ionomer cement kit according to any one of claims 1 to 3, wherein
the (A) water-soluble organic solvent is at least one selected from the group consisting of ethanol, isopropanol and acetone.

5. The dental resin-reinforced glass ionomer cement kit according to any one of claims 1 to 4, wherein
the (F) tri- or more functional (meth)acrylamide-based polymerizable monomer is a compound represented by formula (2): 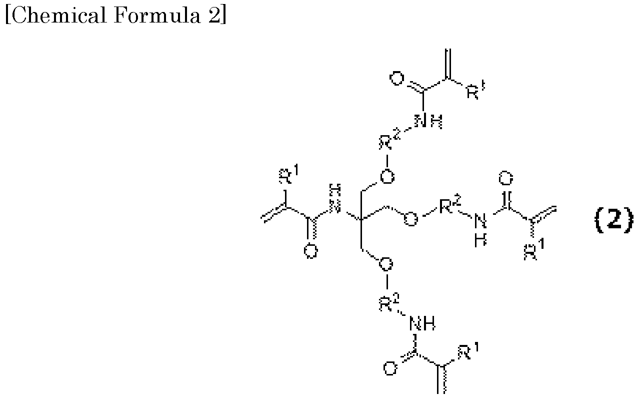 wherein R¹ represents a hydrogen atom or a methyl group and R¹s may be the same or different from each other, and R² represents a linear or branched alkylene group having 2 to 6 carbon atoms which may have a substituent and R²s may be the same or different from each other.

6. The dental resin-reinforced glass ionomer cement kit according to any one of claims 1 to 5, wherein
the dental resin-reinforced glass ionomer cement composition (ii) further contains (I) acidic group-containing polymerizable monomer.

7. The dental resin-reinforced glass ionomer cement kit according to any one of claims 1 to 6, wherein
the dental tooth surface treatment material composition (i) further contains (J) 10-(meth)acryloyloxydecyl dihydrogen phosphate in the range of 0.1 % by mass or more and 10 % by mass or less.

8. The dental resin-reinforced glass ionomer cement kit according to any one of claims 1 to 7, wherein
the dental tooth surface treatment material composition (i) contains (H) polymerization initiator in the range of 0.01% by mass or more and 10 % by mass or less.

9. The dental resin-reinforced glass ionomer cement kit according to any one of claims 1 to 8, wherein
the dental resin-reinforced glass ionomer cement composition (ii) contains (F) tri or more functional (meth)acrylamide-based polymerizable monomer in the range of 0.1% by mass or more and 30 % by mass or less.

10. The dental resin-reinforced glass ionomer cement kit according to claim 2 or 3, wherein
the dental resin-reinforced glass ionomer cement kit further contains (I) acidic group-containing polymerizable monomer.

11. A method for applying the dental resin-reinforced glass ionomer cement kit according to any one of claims 1 to 10 to tooth substance, comprising
a first step in which the dental tooth surface treatment material composition (i) is applied to the tooth surface,
a second step in which the applied surface is dried immediately without washing with water,
a third step in which the dried surface is applied with the dental resin-reinforced glass ionomer cement composition (ii) without irradiating light, and
a fourth step in which the applied dental resin-reinforced glass ionomer cement composition (ii) is hardened irradiating light.
